Europäisches Patentamt

European Patent Office

Office européen des brevets

(11) Publication number: **0 020 147**
**B2**

# NEW EUROPEAN PATENT SPECIFICATION

(45) Date of publication of the new patent specification:
13.01.88

(51) Int. Cl.⁴: **C 12 N 15/00, C 12 P 21/02**

(21) Application number: 80301785.4

(22) Date of filing: 29.05.80

(54) A DNA transfer vector for human pre-growth hormone, a microorganism transformed thereby, and a method of cloning therefor.

(30) Priority: 01.06.79 US 44647

(43) Date of publication of application:
10.12.80 Bulletin 80/25

(45) Publication of the grant of the patent:
25.01.84 Bulletin 84/4

(45) Mention of the opposition decision:
13.01.88 Bulletin 88/2

(84) Designated Contracting States:
AT BE CH DE FR GB IT LI LU NL SE

(56) References cited:
EP-A-0 001 929
DE-A-2 825 595
GB-A-2 031 434
LU-A-79 714

NATURE, vol. 276, 21/28 December 1978, pages 795-798 P.H. SEEBURG et al.: "Synthesis of growth hormone by bacteria"
NATURE, vol. 270, 8th December 1977, pages 486-494 P.H. SEEBURG et al.: "Nucleotide sequence and amplification in bacteria of structural gene for rat growth hormone"
CHEMICAL ABSTRACTS, vol. 89, no. 13, 25th September 1978, page 412, no. 103530q Columbus, Ohio, U.S.A. M.M. HARPOLD et al.: "Construction and identification by positive hybridization-translation of a bacterial plasmid containing a rat growth hormone structural gene sequence"
SCIENCE, vol. 205, 10th August 1979, pages 602-607

(73) Proprietor: THE REGENTS OF THE UNIVERSITY OF CALIFORNIA, 2199 Addison Street, Berkeley California 94720 (US)

(72) Inventor: Baxter, John Darling, 131, San Pablo Avenue, San Francisco California 94127 (US)
Inventor: Goodman, Howard Michael, 3006, Clay Street, San Francisco California 94115 (US)
Inventor: Martial, Joseph Augustin, 38, Sunrise Avenue, Mill Valley California 94941 (US)
Inventor: Hallewell, Robert Alexander, 1504, Masonic Street, San Francisco California 94117 (US)

(74) Representative: De Minvielle- Devaux, Ian Benedict Peter, CARPMAELS & RANSFORD 43, Bloomsbury Square, London WC1A 2RA (GB)

(56) References cited: (continuation)
J.A. MARTIAL: "Human growth hormone: complementary DNA cloning and expression in bacteria"
E. KLEIN/D. REINWEIN, Klinische Endokrinologie, Stuttgart/New York 1978, p. 56-62
M. ALLGOEWER (Hg.), Allgemeine und spezielle Chirurgie, 3. A. Berlin/Heidelberg/New York 1976, p. 540-543
M. REIFFERSCHEID/S. WELLER, Chirurgie, Stuttgart/New York, 1981, Vol. I, S. 209

EP 0 020 147 B2

## Description

The invention relates to a DNA transfer vector comprising a deoxynucleotide sequence coding for human pre-growth hormone. It also relates to a microorganism transformed thereby, and to a method of cloning therefor.

The invention herein provides the isolated and purified (hereinafter "cloned") human gene coding for growth hormone, including a portion of the pre-sequence thereof, a method for isolating and purifying the gene, and a method for transferring the gene to and replicating the gene in a microorganism. The cloned gene is expressed by a host microorganism when fused with a host-expressible procaryotic gene. The gene is useful in the production of human growth hormone for therapeutic purposes.

Growth hormone is produced principally by the anterior lobe of the pituitary gland, also termed the adenohypophysis. The hormone is normally produced throughout life, although in highest amounts during childhood. Although its mechanism of action is not understood in detail, growth hormone is known to affect glucose and lipid metabolism and to promote protein synthesis.

A variety of clinical disorders attributable to growth hormone deficiency are known. Severe cases of pituitary dwarfism are currently treated with human growth hormone isolated from cadavers. Since most non-primate growth hormones are ineffective in humans, there is no suitable alternative source. The supply of material is very small, the procedure for isolating and purifying the hormone is complex and expensive. This is illustrated by the fact that the cost of treatment is estimated in the U.S.A. to be $ 5000.00 per year per patient. Currently, treatment of growth hormone deficiency is limited to the most severe cases. More than 1000 new patients require treatment each year. If an adequate supply of hormone were available at reasonable cost, many thousands of patients with less severe deficiency conditions or with certain other growth problems would be treatable. In addition, experimental evidence indicates that human growth hormone is useful for treatment of gastrointestinal haemorrhage, fracture healing, metabolic bone disease and wound healing.

Growth hormone is a protein. The amino acid sequence of human growth hormone, determined by conventional techniques, is given in Table 1.

## Table 1

```
        1                                    10
NH2-Phe-Pro-Thr-Ile-Pro-Leu-Ser-Arg-Leu-Phe-Asp-Asn-Ala-Met-
                    20
Leu-Arg-Ala-His-Arg-Leu-His-Gln-Leu-Ala-Phe-Asp-Thr-Tyr-
    30                                   40
Glu-Glu-Phe-Glu-Glu-Ala-Tyr-Ile-Pro-Lys-Glu-Gln-Lys-Tyr-
                        50
Ser-Phe-Leu-Gln-Asn-Pro-Gln-Thr-Ser-Leu-Cys-Phe-Ser-Glu-
            60                               70
Ser-Ile-Pro-Thr-Pro-Ser-Asn-Arg-Glu-Glu-Thr-Gln-Gln-Lys-
                        80
Ser-Asn-Leu-Gln-Leu-Leu-Arg-Ile-Ser-Leu-Leu-Leu-Ile-Gln-
                        90
Ser-Try-Leu-Glu-Pro-Val-Glu-Phe-Leu-Arg-Ser-Val-Phe-Ala-
    100                                  110
Asn-Ser-Leu-Val-Tyr-Gly-Ala-Ser-Asn-Ser-Asp-Val-Tyr-Asp-
                        120
Leu-Leu-Lys-Asp-Leu-Glu-Glu-Gly-Ile-Gln-Thr-Leu-Met-Gly-
            130                              140
Arg-Leu-Glu-Asp-Gly-Ser-Pro-Arg-Thr-Gly-Gln-Ile-Phe-Lys-
                        150
Gln-Thr-Tyr-Ser-Lys-Phe-Asp-Thr-Asn-Ser-His-Asn-Asp-Asp-
                        160
Ala-Leu-Leu-Lys-Asn-Tyr-Gly-Leu-Leu-Tyr-Cys-Phe-Arg-Lys-
    170                                  180
Asp-Met-Asp-Lys-Val-Glu-Thr-Phe-Leu-Arg-Ile-Val-Gln-Cys-
                      190 191
Arg-Ser-Val-Glu-Gly-Ser-Cys-Gly-Phe-COOH.
```

Chemical synthesis of this sequence of 191 amino acids is not feasible using conventional techniques. In the pituitary gland, the initial biosynthetic product is termed pre-growth hormone and contains, in addition to the sequence of Table 1, a twenty-six amino acid sequence, termed the signal peptide, attached to the amino-terminal end. The signal peptide sequence is:

-26           -20           -15
$NH_2$-Met-Ala-Thr-Gly-Ser-Arg-Thr-Ser-Leu-Leu-Leu-Ala-Phe-Gly
     -10           -5           -1
Leu-Leu-Cys-Leu-Pro-Trp-Leu-Gln-Glu-Ala-Val-Pro.

The twenty-six amino acid signal peptide is considered to function by conferring the ability to insert and pass through the cell membrane, thereby permitting excretion of the protein from the cell in which it was synthesized (See, Blobel, G. et al., *J. Cell. Biol. 67*, 835 (1975))

Several instances of signal peptides are known for eucaryotic proteins to be transported across membrane barriers. A specific cleavage enzyme has been observed in a cell-free system which hydrolyzes the peptide bond between the signal peptide and the active protein in association with passage through a cell membrane. (See. Blobel, G. et. al., *Proc. Nat. Acad. Sci. USA 75*, 361 (1978)).

Recent advances in biochemistry and in recombinant DNA technology have made it possible to achieve the synthesis of specific proteins under controlled conditions independent of the higher organism from which they are normally isolated. Such biochemical synthetic methods employ enzymes and sub-cellular components of the protein synthesizing machinery of living cells, either *in vitro,* in cellfree systems, or in vivo, in microorganisms. In either case, the key element is provision of a deoxyribonucleic acid (DNA) of specific sequence which contains the information necessary to specify the desired amino acid sequence. Such a specific DNA is herein termed a gene. The coding relationship whereby a deoxynucleotide sequence is used to specify the amino acid sequence of a protein is described briefly, *infra,* and operates according to a fundamental set of principles that obtain throughout the whole of the known realm of living organisms.

A cloned gene may be used to specify the amino acid sequence of proteins synthesized by *in vitro* systems. DNA-directed protein synthesizing systems are well-known in the art, see, e.g., Zubay, G., *Ann. Rev. Genetics 7*, 267 (1973). In addition, single-stranded DNA can be induced to act as messenger RNA *in vitro,* resulting in high fidelity translation of the DNA sequence (Salas, J. et al., *J. Biol. Chem. 243,* 1012 (1968)). Other techniques well known in the art may be used in combination with the above procedures to enhance yields.

Developments in recombinant DNA technology have made it possible to isolate specific genes or portions thereof from higher organisms, such as man and other mammals, and to transfer the genes or fragments to a microorganism, such as bacteria or yeast. The transferred gene is replicated and propagated as the transformed microorganism replicates. As a result, the transformed microorganism may become endowed with the capacity to make whatever protein the gene or fragment encodes, whether it be an enzyme, a hormone, an antigen or an antibody, or a portion thereof. The microorganism passes on this capability to its progeny, so that in effect, the transfer has resulted in a new strain, having the described capability. See, for example, Ullrich, A. et al., *Science 196,* 1313 (1977), and Seeburg, P.H. et al., *Nature 270,* 486 (1977). A basic fact underlying the application of this technology for practical purposes is that DNA of all living organisms, from microbes to man, is chemically similar, being composed of the same four nucleotides. The significant differences lie in the sequences of these nucleotides in the polymeric DNA molecule. The nucleotide sequences are used on specify the amino acid sequences of proteins that comprise the organism. Although most of proteins of different organisms differ from each other, the coding relationship between nucleotide sequence and amino acid sequence is fundamentally the same for all organisms. For example, the same nucleotide sequence which codes for the amino acid sequence of HGH in human pituitary cells, will, when transferred to a microorganism, be recognised as coding for the same amino acid sequence.

Abbreviations used herein are given in Table 2.

**Table 2**

| | | | | |
|---|---|---|---|---|
| DNA- | deoxyribonucleic acid | . | A-Adenine | |
| RNA- | ribonucleic acid | | T-Thymine | |
| cDNA- | complementary DNA | | G-Guanine | |
| | (enzymatically synthesized | | C-Cytosine | |
| | from an mRNA sequence) | | U-Uracil | |
| mRNA- | messenger RNA | | ATP-adenosine triohosphate | |
| dATP- | deoxyadenosine triphosphate | | TTP-thymidine triphosphate | |
| dGTP- | deoxyguanosine triphosphate | | EDTA-Ethylenediaminetetra-acetic acid | |
| dCTP- | deoxycytidine triphosphate | | | |

The coding relationships between nucleotide sequence in DNA and amino acid sequence in protein are collectively known as the genetic code, shown in Table 3.

**Table 3**

Genetic code

| | |
|---|---|
| Phenylalanine (Phe) | TTK |
| Leucine (Leu) | XTY |
| Isoleucine (Ile) | ATM |
| Methionine (Met) | ATG |
| Valine (Val) | GTL |
| Serine (Ser) | QRS |
| Proline (Pro) | CCL |
| Threonine (Thr) | ACL |
| Alanine (Ala) | GCL |
| Tyrosine (Tyr) | TAK |
| Termination signal | TAJ |
| Termination signal | TGA |
| Histidine (His) | CAK |
| Glutamine (Gln) | CAJ |
| Asparagine (Asn) | AAK |
| Lysine (Lys) | AAJ |
| Aspartic acid (Asp) | GAK |
| Glutamic acid (Glu) | GAJ |
| Cysteine (Cys) | TGK |
| Tryptophan (Try) | TGG |
| Arginine (Arg) | WGZ |
| Glycine (Gly) | GGL |

Key: Each 3-letter deoxynucleotide triplet corresponds to a trinucleotide of mRNA, having a 5'-end on the left and a 3'-end on the right. All DNA sequences given herein are those of the strand whose sequence corresponds to the mRNA sequence, with thymine substituted for uracil. The letters stand for the purine or pyrimidine bases forming the deoxynucleotide sequence.

A = adedenine
G = guanine
C = cytosine
T = thymine
X = T or C if Y is A or G
X = C if Y is C or T
Y = A, G, C or T if X is C
Y = A or G if X is T
W = C or A if Z is A or G
W = C if Z is C or T
Z = A, G, C or T if W is C
Z = A or G if W is A
QR = TC if S is A, G, C or T
J = A or G
K = T or C
L = A, T, C or G
M = A, C or T
QR = AG if S is T or C
S = A, G, C or T if QR is TC
S = T or C if QR is AG

An important feature of the code, for present purposes, is the fact that each amino acid is specified by a trinucleotide sequence, also known as a nucleotide triplet. The phosphodiester bonds joining adjacent triplets are chemically indistinguishable from all other internucleotide bonds in DNA. Therefore the nucleotide sequence cannot be read to code for a unique amino acid sequence without additional information to determine the reading frame, which is the term used to denote the grouping of triplets used by the cell in decoding the genetic message.

Many recombinant DNA techniques employ two classes of compounds, transfer vectors and restriction enzymes, to be discussed in turn. A transfer vector is a DNA molecule which contains, *inter alia*, genetic information which insures its own replication when transferred to a host microorganism strain. Examples of transfer vectors commonly used in bacterial genetics are plasmids and the DNA of certain bacteriophages. Although plasmids have been used as the transfer vectors for the work described herein, it will be understood that other types of transfer vectors may be employed. Plasmid is the term applied to any autonomously

4

replicating DNA unit which might be found in a microbial cell, other than the genome of the host cell itself. A plasmid is not genetically linked to the chromosome of the host cell. Plasmid DNA's exist as double-stranded ring structures generally on the order of a few million daltons molecular weight, although some are greater than $10^8$ daltons in molcular weight. They usually represent only a small percent of the total DNA of the cell. Transfer vector DNA is usually separable from host cell DNA by virtue of the great difference in size between them. Transfer vectors carry genetic information enabling them to replicate within the host cell, in most cases independently of the rate of host cell division. Some plasmids have the property that their replication rate can be controlled by the investigator by variations in the growth conditions. Plasmid DNA exists as a closed ring. However, by appropriate techniques, the ring may be opened, a fragment of heterologous DNA inserted, and the ring reclosed, forming an enlarged molecule comprising the inserted DNA segment. Bacteriophage DNA may carry a segment of heterologous DNA inserted in place of certain non-essential phage genes. Either way, the transfer vector serves as a carrier or vector for an inserted fragment of heterologous DNA.

Transfer is accomplished by a process known as transformation. During transformation, bacterial cells mixed with plasmid DNA incorporate entire plasmid molecules into the cells. Although the mechanics of the process remain obscure, it is possible to maximize the proportion of bacterial cells capable of taking up plasmid DNA and hence of being transformed, by certain empirically determined treatments. Once a cell has incorporated a plasmid, the latter is replicated within the cell and the plasmid replicas are distributed to the daughter cells when the cell divides. Any genetic information contained in the nucleotide sequence of the plasmid DNA can, in principle, be expressed in the host cell. Typically, a transformed host cell is recognised by its acquisition of traits carried on the plasmid, such as resistans to certain antibiotics. Different plasmids are recognisable by the different capabilities or combination of capabilities which they confer upon the host cell containing them. Any given plasmid may be made in quantity by growing a pure culture of cells containing the plasmid and isolating the plasmid DNA therefrom.

Restriction endonucleases are hydrolytic enzymes capabie of catalyzing site-specific cleavage of DNA molecules. The locus of restriction endonuclease action is determined by the existence of a specific nucleotide sequence. Such a sequence is termed the recognition site for the restriction endonuclease. Restriction endonucleases from a variety of sources have been isolated and characterized in terms of the nucleotide sequence of their recognition sites. Some restriction endonucleases hydrolyze the phosphodiester bonds on both strands at the same point, producing blunt ends. Others catalyze hydrolysis of bonds separated by a few nucleotides from each other, producing free single stranded regions at each end of the cleaved molecule. Such single stranded ends are self-complementary, hence cohesive, and may be used to rejoin the hydrolyzed DNA. Since any DNA susceptible of cleavage by such an enzyme must contain the same recognition site, the same cohesive ends will be produced, so that it is possible to join heterologous sequences of DNA which have been treated with restriction endonuclease to other sequences similarly treated. See Roberts, R. J., *Crit. Rev. Biochem.* 4, 123 (1976). Restriction sites are relatively rare, however the general utility of restriction endonucleases has been greatly amplified by the chemical synthesis of double stranded oligonucleotides bearing the restriction site sequence. Therefore virtually any segment of DNA can be coupled to any other segment simply by attaching the appropriate restriction oligonucleotide to the ends of the molecule, and subjecting the product to the hydrolytic action of the appropriate restriction endonuclease, thereby producing the requisite cohesive ends. See Heyneker, H. L., et al., *Nature 263,* 748 (1976) and Scheller, R. H., et al., *Science 196,* 177 (1977). An important feature of the distribution of restriction endonuclease recognition sites is the fact that they are randomly distributed with respect to reading frame. Consequently, cleavage by restriction endonuclease may occur between adjacent codons or it may occur within a codon.

More general methods for DNA cleavage or for end sequence modification are available. A variety of non-specific endonucleases may be used to cleave DNA randomly, as discussed *infra.* End sequences may be modified by creation of oligonucleotide tails of dA on one end and dT at the other, or of dG and dC, to create restriction sites without the need for specific linker sequences, when the ends are joined.

The term "expression" is used in recognition of the fact that an organism seldom if ever makes use of all its genetically endowed capabilities at any given time. Even in relatively simple organisms such as bacteria, many proteins which the cell is capable of synthesizing are not synthesized, although they may be synthesized under appropriate environmental conditions. When the protein product, coded by a given gene, is synthesized by the organism, the gene is said to be expressed. If the protein product is not made, the gene is not expressed. Normally, the expression of genes in *E. coli* is regulated as described generally, *infra,* in such manner that proteins whose function is not useful in a given environment are not synthesized and metabolic energy is conserved.

The means by which gene expression is controlled in *E. coli* is well understood, as the result of extensive studies over the past twenty years. See, generally, Hayes, W., *The Genetics of Bacteria and Their Viruses,* 2d edition, John Wiley & Sons, Inc., New York (1968), and Watson, J. D., *The Molecular Biology of the Gene,* 3d edition, Benjamin, Menlo Park, California (1976). These studies have revealed that several genes, usually those coding for proteins carrying out related functions in the cell, are found clustered together in continuous sequence. The cluster is called an operon. All genes in the operon are transcribed in the same direction, beginning with the codons coding for the N-terminal amino acid of the first protein in the sequence and continuing through to the C-terminal end of the last protein in the operon. At the beginning of the operon, proximal to the N-terminal amino acid codon, there exists a region of the DNA, termed the control region, which includes a variety of controlling elements including the operator, promoter and sequences for the ribosomal binding sites. The function of these sites is to permit the expression of those genes under their control to be responsive to the needs of the organism. For example, those genes coding for enzymes required exclusively for utilization of lactose are normally not appreciably expressed unless lactose or an analog thereof is actually present in the medium. The control region functions that must be present for expression to occur are the initiation of transcription and the initiation of translation. Expression of the first gene in the sequence is initiated by the initiation of transcription and translation at the position coding for the N-terminal amino acid of the first protein of the operon. The expression of each gene downstream from that point is also initiated in turn, at least until a termination signal or another operon is encountered with its own control region, keyed to respond to a different set of environmental cues. While there are many variations in detail on this general scheme, the important fact is that, to be expressed in a procaryote such as *E. coli,* a gene must be properly located with respect to a control region having initiator of transcription and initiator of translation functions.

It has been demonstrated that genes not normally part of a given operon can be inserted within the operon and controlled by it. The classic demonstration was made by Jacob, F., et al., *J. Mol. Biol. 13,* 704 (1965). In that experiment, genes coding for enzymes involved in a purine biosynthesis pathway were transferred to a region controlled by the lactose operon. The expression of the purine biosynthetic enzyme was then observed to be repressed in the absence of lactose or a lactose analog, and was rendered unresponsive to the environmental cues normally regulating its expression.

In addition to the operator region regulating the initiation of transcription of genes downstream from it, there are known to exist codons which function as stop signals, indicating the C-terminal end of a given protein. See Table 2. Such codons are known as termination signals and also as nonsense codons, since they do not normally code for any amino acid. Deletion of a termination signal between structural genes of an operon creates a fused gene which could result in the synthesis of a chimeric protein consisting of two amino acid sequences coded by adjacent genes, joined by a peptide bond. That such chimeric proteins are synthesized when genes are fused was demonstrated by Benzer, S., and Champe, S. P., *Proc. Nat. Acad. Sci. USA 48,* 114 (1962).

Once a given gene has been isolated, purified and inserted in a transfer vector, the overall result of which is termed the cloning of the gene, its availability in substantial quantity is assured. The cloned gene is transferred to a suitable microorganism, wherein the gene replicates as the microorganism proliferates and from which the gene may be reisolated by a conventional means. Thus is provided a continuously renewable source of the gene for further manipulations, modifications and transfers to other vectors or other loci within the same vector.

Expression is obtained by transferring the cloned gene, in proper orientation and reading frame, into a control region such that read-through from the procaryotic gene results in synthesis of a chimeric protein comprising the amino acid sequence coded by the cloned gene. A variety of specific protein cleavage techniques may be used to cleave the chimeric protein at a desired point so as to release the desired amino acid sequence, which may then be purified by conventional means. Techniques for constructing an expression transfer vector having the cloned gene in proper juxtaposition with a control region are described in Polisky, B., et al., *Proc. Nat. Acad. Sci. USA 73,* 3900 (1976); Itakura, K. et al., *Science 198,* 1056 (1977); Villa-Komaroff, L., et al., *Proc. Nat, Acad. Sci. USA 75,* 3727 (1978); Mercereau-Puijalon, O., et al., *Nature 275,* 505 (1978); Chang, A. C. Y. et al, *Nature 275,* 617 (1978), and in U.S. Application Serial No. 933,035 by Rutter, et al., said application incorporated herein by reference as though set forth in full.

In summary, the process whereby a mammalian protein, such as human pre-growth hormone, is produced with the aid of recombinant DNA technology first requires the cloning of the mammalian gene. Once cloned, the gene may be produced in quantity, further modified by chemical or enzymic means and transferred to an expression plasmid. The cloned gene is also useful for isolating related genes, or, where a fragment is cloned, for isolating the entire gene, by using the cloned gene as a hybridization probe. Further the cloned gene is useful in proving by hybridization, the identity of homology of independent isolates of the same or related genes. Because of the nature of the genetic code, the cloned gene, when translated in the proper reading frame, will direct the production only of the amino acid sequence for which it codes and no other.

The cloned gene for human pre-growth hormone is useful in a variety of ways. Transposition to an expression transfer vector will permit the synthesis of pre-growth hormones by a host microorganism transformed with the vector carrying the cloned gene. Growth of the transformed host will result in synthesis of pre-growth hormone as part of a chimeric protein. If the procaryotic portion of the chimeric protein is the signal portion of an excreted or otherwise compartmentized host protein, excretion or compartmentization can occur, greatly enhancing the stability and ease of purification of the pre-growth hormone chimera. Additionally, where the procaryotic portion is short, excretion from the procaryotic host will be facilitated by the pre-sequence itself, if the pre-sequence functions in the procaryotic host as it does in the eucaryotic cell. The cloned gene is further useful in hybridization techniques for the isolation of genetic material having partial sequence homology. The growth hormone genes of other mammalian species are isolatable in this manner since, despite their lack of physiological cross-reactivity, sufficient sequence similarly exists for effective hybridization. The growth hormones of various animal species are useful for agricultural and veterinary purposes. The growth hormone gene of an individual human patient is isolatable by the hybridization technique, for the purpose of analysis and treatment of a specific growth disorder.

The cloned pre-growth hormone gene can be used, in a variety of techniques for the production of pre-growth hormone. Pre-growth hormone itself is useful because it can be converted to growth hormone by known enzymatic and chemical techniques. For example, the pre-sequence can be removed by a soluble enzymic preparation as described by Blobel, G. *supra,* specific for removal of signal peptides.

As disclosed herein, a cDNA having a base sequence coding for human pre-growth hormone has been cloned. The structure of the cloned cDNA has been verified by nucleotide sequence analysis.

The original source of genetic material is human pituitary tumor tissue obtained by surgery. Preferably, genetic material is isolated from the cells of an individual human, and a special technique is disclosed for cloning the gene from individual human tumors.

Messenger RNA was isolated from the cells and partially purified by chromatography and sedimentation. Active fractions were identified by their ability to direct the synthesis of a protein of approximately 200 amino acids in a cell-free protein synthesis system, as judged by gel electrophoresis.

DNA complementary to the isolated messenger RNA (cDNA) was synthesized using reverse transcriptase, in two reaction cycles to generate double-stranded cDNA, as described in detail, *infra.* The heterogeneous, reverse transcriptase double-stranded DNA reaction product was fractionated according to length by gel electrophoresis. DNA migrating in a region corresponding to about 800 base pairs in length was chosen for further study. The major portion of the 800 base-pair cDNA was shown to be that coding for growth hormone by separately treating it with the restriction endonucleases *PvuII* and *BgII. PvuII* is known to cleave growth hormone cDNA to yield an approximately 495 base-pair fragment. *BgII* is known to have one cleavage site within this fragment. After cleavage and fractionation the anticipated bands characteristic of growth hormone cDNA were obtained.

The uncleaved, heterogeneous, double-stranded cDNA of approximately 800 base-pairs length was treated to provide specific linker oligonucleotide sequences at each end, to facilitate insertion into a site of the same restriction specificity on a transfer vector.

For cloning, a transfer vector providing good selection and stable replication properties was selected. The treated, 800 base-pair cDNA was inserted into a transfer vector at a predetermined site on the vector DNA to form a recombinant transfer vector, using currently available techniques. Host microorganism cells were transformed with the recombinant vector. Transformants were selected according to the criteria established for insertion at the predetermined site. Single colonies, each derived from a single transformed microorganism cell, were picked and grown in individual cultures to permit replication of the recombinant transfer vector DNA clones. Transfer vector DNA was then isolated and run on slab gel electrophoresis runs used to screen for transfer vectors of the correct size and to eliminate defects, such as deletions. Colonies yielding recombinant transfer vector DNA of appropriate size were grown in larger individual cultures to isolate transfer vector DNA and subject the DNA to restriction enzyme analysis. The DNA inserted in the transfer vector could be identified, for each clone, by treatment with the restriction enzyme used to cleave at the insertion site, and by the enzymes *PvuII* and *BgII,* with which growth hormone cDNA yields a characteristically sized fragment. A clone satisfying all requirements was selected for definitive identification of the nucleotide sequence of the cloned pre-growth hormone gene, and for transfer to an appropriate expression plasmid.

Growth hormone is synthesized exclusively by the anterior lobe of the pituitary gland in normal individuals. In principle, the gene for growth hormone could be isolated from any tissue. However, there are intervening sequences in the human growth hormone gene which, in the normal eucaryotic environment, are transcribed and then processed out to yield the "mature" mRNA without intervening sequences. A deoxynucleotide sequence translatable in a procaryotic cell, such as a bacterium may not contain such intervening sequences, since procaryotic cells would be expected to translate such sequences giving rise to an undesired protein product. There, as a practical matter, the deoxynucleotide sequence must be derived from messenger RNA isolated from cells actively synthesing growth hormone.

The general outline for cloning a cDNA coding for a given protein, starting from eucaryotic messenger RNA has been described by Ullrich, A., *supra,* and Seeburg, P. H., et al., *supra.* Briefly recapitulated herein, the procedure includes the following steps:

1) isolation of polyadenulated RNA, substantially undegraded, from the differentiated cells or tissue that produce the desired protein,

2) synthesis of a DNA strand complementary in base sequence to the isolated RNA, using reverse transcriptase and yielding an RNA-DNA hybrid,

3) selective degradation of the RNA portion of the hybrid, leaving a single-stranded cDNA,

4) synthesis of DNA complementary to the single-stranded cDNA, to form double-stranded cDNA,

5) fractionation of the double-stranded cDNA, to enrich the preparation for molecules in the desired size range,

6) preparation of a selected transfer vector to permit insertion of the cDNA,

7) covalent joining of the cDNA with the transfer vector, using DNA ligase, to yield a recombinant transfer vector, and

8) replication of the desired recombinant transfer vector by transformation, selection and growth of a suitable host microorganism strain.

Anterior pituitary cells may be obtained either from human pituitary glands removed by transphenoidal hypophysectomy or from certain human pituitary tumor cell lines grown in culture. RNA isolated from either source is partially purified to enhance the ultimate yield and used as a template for transcription into cDNA. Partial purification may include sedimentation in a sucrose gradien to remove RNA that is either too small or too large. Additional purification by chromatography on oligo-dT cellulose may be employed to isolate polyadenylated RNA, which is the principal form of messenger RNA in eucaryotic cells.

Partially purified messenger RNA may be used as a template to construct cDNA using the enzyme, reverse transcriptase. The formation of double stranded cDNA, using reverse transcriptase in combination with S1 nuclease and certain separation techniques, has been previously described. (See. Ullrich, A. et. al., *Science* 195, 1313 (1977) and Seeburg, P. H. et al., *Nature 270*, 486 (1977)). The resulting cDNA product is heterodisperse in length. If the nucleotide sequence of the cDNA to be cloned were known or predictable within reasonable probability limits, and if restriction sites could be located reasonably close to the beginning and end of the gene to be cloned, suitable restriction enzymes could be used to render the desired cDNA product homogeneous in length and separable from contaminants of differing sequences. It will be understood that such nucleotide sequence information is unavailable for genes, such as human pre-growth hormone, which have never previously been isolated and purified, except possibly under certain exceptional and fortuitous circumstances. Consequently, all subsequent steps generally must be carried out with the heterogeneous cDNa product.

Sufficient information regarding part of the nucleotide sequence of human growth hormone cDNA was available from previous data disclosed in Application Serial, No. 897,710 by Goodman, et al., and in corresponding LU-A-79714 to make possible the idencification of subfragments of the pregrowth hormone gene, as described, *supra*. From the known number of amino acids in human pre-growth hormone, the approximate length of the desired cDNA was estimated to be 800 base pairs. Double-stranded cDNA whose gel-electrophoresis mobility corresponded to about 800 base pairs was found, upon cleavage with the restriction enzymes *PvuII* and *BglII*, to contain predominantly growth hormone coding sequences.

The transfer of cDNA to a DNA transfer vector could be accomplished at any random site on the vector generated by a single endonuclease hit coupled with attachment of the cDNA by DNA ligase-catalyzed blunt-end ligation. (Sgaramella, V. et al., *Proc. Nat. Acad. Sci. USA 67*, 1468 (1970)). However, selection is greatly simplified and yields are enhanced by using a specific insertion site whose location in the transfer vector is known, and by treating the cDNA to enhance the insertion specificity. In a preferred embodiment, a plasmid transfer vector having a single restriction site located with a marker gene is used. The circular plasmid DNA is cut with the appropriate restriction endonuclease. Joining the ends of the cDNA to the ends of the open plasmid DNA results in formation of a circular, recombinant plasmid, with the cDNA inserted at the restriction site, effectively interrupting the marker gene, thereby causing loss of the marker gene function. For example, plasmid pBR322 contains a single *HindIII* restriction site, located with the promoter for the gene for tetracycline resistance. *E. coli* cells transformed by pBR322 are rendered able to grow and divide in the presence of as much as 20 µg/ml tetracycline in the growth medium. In contrast, under the same condition, untransformed cells are killed. Cells transformed by a recombinant pBR322 containing an insertion at the *HindIII* site are unable to grow and divide in the presence of 20 µg/ml tetracycline. The plasmid pBR322 also carries a gene conferring ampicillin resistance. These genetic markers in combination permit selection of transformed cells from untransformed cells by growth of only the former in the presence of ampicillin, and selection of those transformed by non-recombinant pBR322 from recombinant pBR322 by growth of only the former in the presence of tetracycline. Insertion specificity and yield in *HindIII*-cut pBR322 is enhanced by attachment of *HindIII*-linker oligonucleotides to the ends of the heterogeneous cDNA. See Scheller, R. H. et al., *supra*. Further side reaction in formation of the recombinant transfer vector, such as ring closure without insertion of the cDNA, or dimerization, are substantially reduced by pretreating the ends of restriction-cut transfer vector DNA to remove the 5′ terminal phosphate groups, as described by Shine in co-pending application Serial No. 898,887 (LU-A-79714). Used in combination, the foregoing techniques substantially increase the absolute and relative yields of the desired recombinant transfer vectors.

In previous cloning studies, the cDNA was synthesized from messenger RNA derived from pooled cell extracts of several individuals. The present invention provides techniques for obtaining a cloned gene derived from a single individual. The technique combines reductions in scale and reaction volume, assays that do not consume the product, and elimination of carrier DNA. The latter feature surprisingly improves over-all yield and efficiency of the various enzyme-catalyzed reactions used in the process. Messenger RNA is isolated from individual pituitary tumors by appropriately scaled published procedures (Seeburg, et al., *supra*). Column chromatography steps may be conducted in small glass tubes with a drawn tip, for example in Pasteur pipettes. Purifications to remove protein may be carried out by any of several suitable phase separation techniques such as phenol extraction, preferably by extraction with chloroform-saturated phenol. Concentration of nucleic acids by precipitation with alcohol at low temperature may be employed throughout, preferably by ethanol precipitation at -70°C. Both the extraction procedure and the precipitation procedure may be accomplished in a single tube, preferably a conical plastic centrifuge tube of 1 ml-2 ml total volume.

Enzyme reactions, such as treatment with reverse transcriptase, may also be carried out in the same small reaction tubes. Typically, the rate and extent of synthesis of single-stranded and double-stranded cDNA is followed by incorporation of a radioisotope label in the nascent cDNA. A convenient label is [322P]incorporated at the alpha position of one of the nucleoside triphosphate precursors, for example $\alpha$-32P-dCTP. Use of radioactive labeling also permits subsequent monitoring of the cDNA product during passage through columns and on electrophoresis gels. Where it is necessary to monitor a series of column chromatography fractions, for example after chromatography to remove unreacted precursors in the reverse transcriptase reaction, a radioassay which does not consume the reaction product is highly desirable. In the present method, fractions collected in the small plastic tubes described, *supra*, are tightly capped. The tubes are then placed directly into scintillation vials without scintillation fluid. The resulting Cerenkov radiation may be counted with reasonable efficiency with appropriate instrument settings.

Procedures involving small amounts of DNA have typically been performed with carrier DNA added to the mixture. The rationale for using DNA is to improve the recovery of the desired DNA, which might otherwise be low due to non-specific adsorption to container walls, or to lack of whatever co-operate effects operate in DNA precipitations and the like. Suitable yields of DNA are obtained in the absence of added carrier DNA, even with the nanogram quantities of DNA available. Omission of carrier DNA poses two distinct advantages in the present method; first, there is no non-specific DNA to yield non-specific reaction products in the enzyme-catalyzed reactions; second, there is no contaminating DNA to yield non-specific recombinant transfer vectors.

By the use of the described techniques in combination, it is possible to clone the deoxynucleotide sequence coding for human pre-growth hormone from the pituitary gland of an individuel human patient. In the following examples, the isolation and purification processes are described in detail and proof of the identity of the cloned gene is given by nucleotide sequence analysis.

## Example 1

Process of cloning a gene coding for human pre-growth hormone from the tissue of a single individual

The pituitary glands of six human patients with pituitary tumors were removed by transphenoidal hypophysectomy, quick-frozen and stored in liquid nitrogen. In the following procedure, each pituitary gland was treated separately, although all 6 were worked up at the same time. The glands were thawed and homogenized in 4 M guanidinium thiocyanate containing 1 M mercaptoethanol buffered to pH 5.0 at 4°C. Each homogenate was layered over 1.2 ml 5.7 M CsCl containing 10 mM EDTA and centrifuged for 18 hours at 37,000 rpm in the SW 50.1 rotor of a Beckman ultracentrifuge at 15°C (Beckman Instrument Co., Fullerton, California). RNA traveled to the bottom of the tube. Further purification of the messenger RNA, using an oligo-dT cellulose column was performed essentially as described by Ullrich, A. et al., *supra*, and Seeburg, P. H. et al., *supra*.

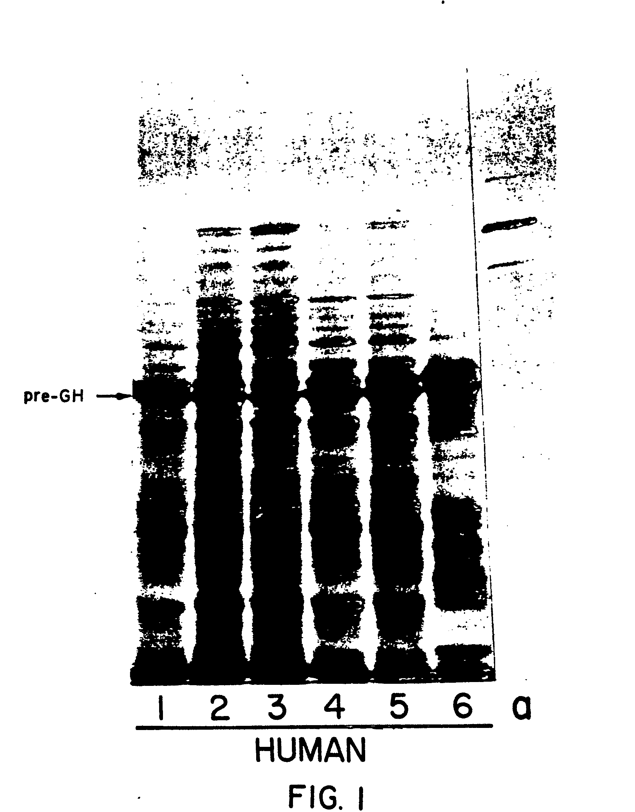

The biological activity of the partially purified messenger RNA was measured by its ability to direct the incorporation of [$^{35}$S]-methionine into growth hormone in a cell-free protein synthesis system derived from wheat germ. The experimental conditions for the test were essentially as described by Martial, J. H. et al., *Proc. Nat. Acad. Sci. USA 74*, 1816 (1977). Figure 1 shows the results of one such test. The radioactive protein products were fractionated by gel electrophoresis on a 12.5 % polyacrylamide gel containing 0.1 % sodium dodecylsulfate for 4 hours at 20 mA. The radioactive protein bands were detected by autoradiography. Band (a) shows [$^{14}$C]-labeled marker proteins from bacteriophage T4 used as size markers. Bands 1-6 show the translation products of the oligo-dT cellulose-purified messenger RNA from the individual tumors. It can be seen with tumors 1-5 that pre-growth hormone constitutes the major cell-free synthetic product.

The RNA preparations most enriched in pre-growth hormone cell-free translational activity (samples producing bands 2, 4 and 5) were pooled and were used as template for the synthesis of double-stranded cDNA. All reaction steps were carried out in 1.5 ml capacity plastic centrifuge tubes. The first reverse transcriptase-catalyzed reaction, resulting in an RNA-DNA hybrid, was carried out in a reaction mixture containing 68 µl of the RNA preparation containing approximately 13 µg RNA, avian myeloblastosis virus reverse transcriptase, 4 µl of undiluted preparation as obtained from the Office of Program Resources and Logistics, Viral Cancer Program, Viral Oncology, Division of Cancer Cause and Prevention, National Cancer Institute, Bethesda, Maryland, and 32 µl of a reaction mixture comprising 10 µl "reverse transcriptase buffer" (0.5 M Tris-HCl pH.8.3, 1 mM EDTA, 70 mM MgCl$_2$, 200 mM KCl), 1 µl 2-mercaptoethanol 1 M, 5 µl oligo-dT 1 µg/ml, 5 µl each of dATP, dGTP and TTP (20 mM stock solutions), 1 µl dCTP 20 mM stock solution, and approximately 2 x 10$^6$ cpm α-[$^{32}$P]-dCTP dissolved directly in the reaction mixture.

The reaction was carried out under conditions essentially as described by Seeburg, P. H. et al., *supra*. The incubation mixture was extracted with chloroform-saturated phenol in the same reaction tube, then precipitated with ethanol, the tubes stored at -70°C for at least 15 minutes, then centrifuged.

The precipitated RNA-DNA hybrid was dissolved in 0.1 N NaOH, 5 mM EDTA, 125 µl, and incubated at 68°C for 1/2 hour to permit selective alkaline hydrolysis of RNA. The reaction mixture was loaded on a small column of Sephadex G-50 in a Pasteur pipette and eluted with buffer containing 10 mM Tris (pH 7.5) and 0.5 mM EDTA. (Sephadex G-50 is a Trade Mark of Pharmacia Inc., Piscataway, New Jersey). The radioactive single-stranded DNA was recovered in a single 2-drop fraction collected in an Eppendorf tube. The fraction tubes were capped, placed in scintillation vials and counted in a liquid scintillation counter set for detection of Cerenkov radiation.

The synthesis of the double-stranded cDNA from the single-stranded cDNA product of the previous reaction steps was carried out by a reverse-transcriptase catalyzed reaction. The reaction was carried out in a total volume of 51 µl containing 30 µl of the single-stranded DNA solution, 3.5 µl of the enzyme solution, and 17.5 µl of a reaction mixture composed as follows: water, 96 µl; reverse-transcriptase buffer, 60 µl; 2-mercaptoethanol 1 M, 6 µl, dATP 20 mM, 15 µl; dGTP 20 mM, 15 µl; TTP 20 mM, 15 µl; dCTP 20 mM, 3 µl; α[$^{32}$P] dCTP, approximately 6 x 10$^8$ cpm, dissolved directly in the reaction mixture. The reaction was allowed to proceed for 90 minutes at 47°C. The reaction was terminated and protein removed by the extraction and Sephadex G-50 chromatography steps described, *supra,* followed by ethanol precipitation as described.

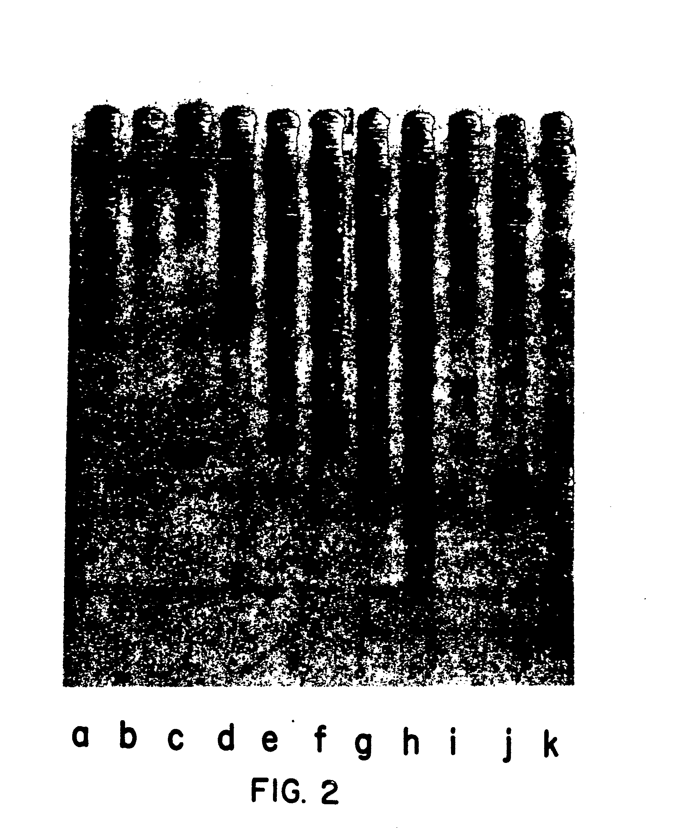

To confirm the fact that cDNA coding for growth hormone was an abundant species in the preparation, a portion of the double-stranded cDNA preparation was cleaved with restriction endonucleases and analyzed by polyacrylamide (4.5 %) gel electrophoresis (Figure 2). Lanes (c) and (k) of Figure 2 show the bands from radiolabeled bacteriophage fd DNA cleaved by endonucleases *Hpa*ll, lane (c), and *Hae*lll, lane (k). Lanes (a) and (b) show the uncleaved double-stranded cDNA. Lanes (d) through (j) show the DNA cleaved by: lane (d) *Pst*l plus *Bgl*ll; lane (e) *Pvu*ll; lane (f) *Pvu*ll plus *Bgl*ll; lane (g) *Hinf* plus *Sma*l; lane (h) *Hae*lll; lane (i) *Pvu*ll; and lane (j) *Hae*lll. The pattern of bands observed was that expected from cDNA complementary to human growth hormone mRNA as based on the structure of a previously-cloned 550 base-pair fragment of human growth hormone, described in application Serial No. 897,710, incorporated herein by reference. These data confirmed the fact that the predominant cDNA was indeed complementary to human growth hormone messenger RNA.

The resulting double-stranded cDNA was treated with S1 nuclease essentially as described by Ullrich, A. et al., *supra,* to cleave the "hairpin" structure in the unpaired loop region. The enzyme treatment was followed, as in previous steps, by extraction, chromatography and precipitation. Any unpaired ends were completed with a DNA polymerase I-catalyzed reaction. The reaction mixture, 20 µl total volume, contained 60 mM Tris-HCl, pH 7.5, 8 mM $MgCl_2$, 10 mM 2-mercaptoethanol, 1 mM ATP, 200 µM each of dATP, dTTP, dGTP and dCTP. The mixture was incubated with 1 unit of *E. coli* DNA polymerase 1 (Boehringer-Mannheim Biochemicals, Indianapolis, Indiana) at 10°C for 10 minutes to exonucleolytically remove any 3'-protruding ends and to fill any 5'-protruding ends with complementary sequences. The reaction was designed to yield a population of cDNA molecules with blunt ends.

Linker oligonucleotides specific for the *Hind*lll restriction site sequence were added to the cDNA ends by blunt end ligation, using DNA ligase, as described by Seeburg, P. H. et al., *supra,* Plasmid pBR322, Bolivar, F. et al., *Gene 2,* 95 (1977) was chosen as transfer vector, for reasons described, *supra.* The insertion and screening techniques have been described by Ullrich, A. et al., *supra.* Ampicillin resistant colonies were picked and transferred to plates containing ampicillin and tetracycline. Those colonies found to be ampicillin-resistant but tetracycline-sensitive were chosen for further study.

Defective insertions, including deletions and duplications, were detected by slab gel electrophoresis runs of *Hind*lll-treated plasmid DNA isolated from cultures grown up from single colonies of transformed cells, selected as described. Such defects were readily detectable by the sizes of the *Hind*lll fragments.

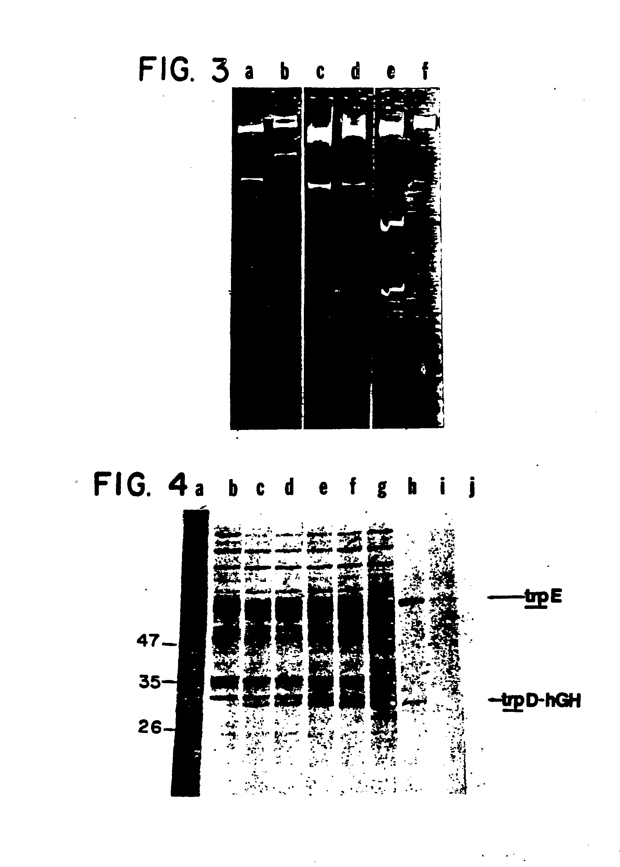

The cloned DNA was identified as coding for human pre-growth hormone, after being released from the transfer vector by *Hind*lll digestion, by further treatment with restriction enzymes *Pvu*ll and *Bgl*ll. The released cDNA and restriction fragments were fractionated by gel electrophoresis, in which the DNA was detected by fluorescence staining with ethidium bromide. The results are shown in Figure 3, oriented such that larger DNA fragments are located nearer the top of the figure. Lane (a) is a previously cloned 550 base-pair fragment of human growth hormone DNA, described in Application Serial No. 897,710. Lane (b) is the 800 base-pair human pre-growth hormone DNA of the present example. Lanes (c) and (e) are samples of the 500 base-pair fragment cleaved with *Pvu*ll and *Bgl*ll, respectively. Lanes (d) and (f) are samples of the 800 base-pair pre-growth hormone DNA cleaved by *Pvu*ll and *Bgl*ll, respectively. Cleavage by *Pvu*ll yields fragments of similar size in both cases, as expected from the known location of a *Pvu*ll site near an end of the 500 base-pair fragment. Cleavage of *Bgl*ll yields larger fragments with the 800 base-pair DNA than with the 550 base-pair fragment, as expected. These findings are consistent with the identification of the cloned 800 base-pair fragment as containing human growth hormone gene sequences. The cloned DNA, designated cHGH800 was chosen for sequence analysis.

## Example 2

Nucleotide sequence analysis of cHGH800

The nucleotide sequence of cHGH800 was determined by the method of Maxam and Gilbert, *Proc. Nat. Acad. Sci. USA 74,* 560 (1977) and by the method of Sanger, F. et al., *Proc. Nat. Acad. Sci. USA 74,* 5463 (1977). The result is shown in Table 4. The cloned gene includes the entire sequence coding for human pre-growth hormone, together with a 29 base-pair region at the 5'-end presumably untranslated, as well as an untranslated region of 108 base pairs at the 3'-end (excluding the poly-A portion). (The 5'- and 3'-ends are designated by reference to that strand of the cDNA corresponding in sequence to the growth hormone messenger RNA).

Some uncertainty remains in the pre-sequence near the beginning of the growth hormone sequence. It is also noted that the nucleotide sequence of Table 4 yields an amino acid sequence for growth hormone which differs slightly from the published sequence. The amino acid sequence inferred from nucleotide sequence analysis is considered to be more likely the correct one. The disagreements occur in distinguishing amino acid pairs such as Gln or Glu, which are less readily distinguishable by direct amino acid sequencing than by nucleotide sequencing. Further, nucleotide sequence analysis provides information previously unobtainable, for example, the probable amino acid sequence of the growth hormone pre-sequence.

## Table 4

Translation of cHGH800

```
                                                      -26
                                                      met  ala  thr  gly  ser  arg
GG   A:C  CUG  UGG  ACA  GCU  CAC  CUA  GCU  GCA      AUG  GCU  ACA  GGC  UCC  CGG
-20                                                   -10
thr  ser  leu  leu  leu  ale  phe  gly  leu  leu      cys  leu  pro  trp
ACG  UCC  CUG  CUC  CUG  GCU  UUU  GGC  CUG  CUC      UGC  CUG  CCC  UGG
                                   1                                            10
leu  gln  glu  ala  val  pro  phe  pro  thr  ile      pro  leu  ser  arg  leu  phe
CUU  CAA  GAG  GCA  GUG  CCU  UUC  CCA  ACC  AUU      CCC  UUA  UCC  AGG  CUU  UUU
                                             20
asp  asn  ala  met  leu  arg  ala  his  arg  leu      his  gln  leu  ala
GAC  AAC  GCU  AUG  CUC  CGC  GCC  CAU  CGU  CUG      CAC  CAG  CUG  GCC
                             30                                                 40
phe  asp  thr  tyr  gln  glu  phe  glu  glu  ala      tyr  ile  pro  lys  glu  gln
UUU  GAC  ACC  UAC  CAG  GAG  UUU  GAA  GAA  GCC      UAU  AUC  CCA  AAG  GAA  CAG
                                             50
lys  tyr  ser  phe  leu  gln  asn  pro  gln  thr      ser  leu  cys  phe
AAG  UAU  UCA  UUC  CUG  CAG  AAC  CCC  CAG  ACC      UCC  CUC  UGU  UUC
                             60                                                 70
ser  glu  ser  ile  pro  thr  pro  ser  asn  arg      glu  glu  thr  gln  gln  lys
UCA  GAG  UCU  AUU  CCG  ACA  CCC  UCC  AAC  AGG      GAG  GAA  ACA  CAA  CAG  AAA
                                             80
ser  asn  leu  glu  leu  leu  arg  ile  ser  leu      leu  leu  ile  gln
UCC  AAC  CUA  GAG  CUG  CUC  CGC  AUC  UCC  CUG      CUG  CUC  AUC  CAG
                             90                                                 100
ser  trp  leu  glu  pro  val  gln  phe  leu  arg      ser  val  phe  ala  asn  ser
UCG  UGG  CUG  GAG  CCC  GUG  CAG  UUC  CUC  AGG      AGU  GUC  UUC  GCC  AAC  AGC
                                             110
leu  val  tyr  gly  ala  ser  asp  ser  asn  val      tyr  asp  leu  leu
CUG  GUG  UAC  GGC  GCC  UCU  GAC  AGC  AAC  GUC      UAU  GAC  CUC  CUA
                             120                                                130
lys  asp  leu  glu  glu  gly  ile  gln  thr  leu      met  gly  arg  leu  glu  asp
AAG  GAC  CUA  GAG  GAA  GGC  AUC  CAA  ACG  CUG      AUG  GGG  AGG  CUG  GAA  GAU
                                             140
gly  ser  pro  arg  thr  gly  gln  ile  phe  lys      gln  thr  tyr  ser
GGC  AGC  CCC  CGG  ACU  GGG  CAG  AUC  UUC  AAG      CAG  ACC  UAC  AGC
                             150                                                160
lys  phe  asp  thr  asn  ser  his  asn  asp  asp      ala  leu  leu  lys  asn  tyr
AAG  UUC  GAC  ACA  AAC  UCA  CAC  AAC  GAU  GAC      GCA  CUA  CUC  AAG  AAC  UAC
                                             170
gly  leu  leu  tyr  cys  phe  arg  lys  asp  met      asp  lys  val  glu
GGG  CUG  CUC  UAC  UGC  UUC  AGG  AAG  GAC  AUG      GAC  AAG  GUC  GAG
                             180                                                190
thr  phe  leu  arg  ile  val  gln  cys  arg  ser      val  glu  gly  ser  cys  gly
ACA  UUC  CUG  CGC  AUC  GUG  CAG  UGC  CGC  UCU      GUG  GAG  GGC  AGC  UGU  GGC
191
phe  AM
UUC  UAG  CUG  CCC  GGG  UGG  CAU  CCU  GUG  ACC      CCU  CCC  CAG  UGC
CUC  UCC  UGG  CCC  UGG  AAG  UUG  CCA  CUC  CAG      UGC  CCA  CCA  GCC  UUG  UCC
UAA  UAA  AAU  UAA  GUU  GCA  UCA  AAA  AAA  AAA
```

## Example 3

Construction of plasmid ptrpED50-HGH

A plasmid designated p*trp*ED5-1, (obtained from Searle Research Laboratories, High Wycombe, Bucks, England), was modified to permit insertion and expression of the cloned pre-growth hormone cDNA. Plasmid p*trp*ED5-1 was derived from the *Hind*III fragment of the *E. coli* tryptophan (trp) operon, transferred from bacteriophage λ*trp*E, as described by Hopkins, A. S. et al., *J. Mol. Biol. 107,* 549 (1976), inserted in the *Hind*III site of pBR322, Bolivar, F. et al., *Gene 2,* 95 (1977).

Plasmid p*trp*ED5-1 was cleaved with *Hin*dlil, and treated with the Kenow fragment of DNA polymerase I to pair the single-stranded 5'-ends (Seeburg, P. H. et al., *supra*). Decanucleotide *Hin*dlll linkers were attached to the plasmid DNA. Cohesive ends were then generated by *Hin*dlll and the plasmid DNA was isolated by Sephadex G100 chromatography. (Sephadex G-100 is a Trade Mark of Pharmacia Inc., Uppsala, Sweden). The circular plasmid was regenerated with the use of T4-DNA ligase, and used to transform the RR-I strain of *E. coli*. Plasmid p*trp*ED50 containing clones were isolated by selection for ampicillin resistance.

Cloned human pre-growth hormone DNA (cHGH800 as described in Examples 1 and 2) was inserted at the *Hin*dlll site of p*trp*ED50. The recombinant transfer vector was used to transform *E. coli* x 1776. Transformants were selected on ampicillin-containing plates. Plasmid DNA isolated from several clones was tested for correct orientation of insertion by cleavage with *Bam*HI and *Ps*tI. Correct orientation, such that codons of pre-growth hormone would be read in phase with those of the *trp* operon, was inferred from the asymmetric location of the *Ps*tI site, yielding a 700 base-pair fragment. Incorrect orientation would yield a 290 base-pair fragment. Plasmids with correct orientation, designated p*trp*ED50-HGH, were then transformed into *E. coli trp*OE ∇ 1.

## Example 4

Expression of human pre-growth hormone

In plasmid p*trp*ED50-HGH, expression of the *trp*E and *trp*D genes is under tryptophan control, such that the levels of protein products coded by these genes is elevated from a basal level by the presence of an inducer such as β-indolylacrylic acid. Induction of *E. coli trp*OE ∇ 1/p*trp*ED50-HGH is expected to result in increased production of the *trp*E gene product and of a *trp*D-pre-growth hormone fusion protein. The latter comprises part of the $NH_2$-terminal portion, the *trp*D gene product joined to human pre-growth hormone.

A culture of *E. coli trp*OE ∇ 1/p*trp*ED50-HGH was induced with β-indolylacrylic acid, and 3 ml samples were pulse labeled with 2 μCi of $^{14}$C-labeled amino acids for a constant time, at various intervals after induction. Samples from the zero and 4 hour induced cultures were immunoprecipitated using formaldehyde-treated *Staphyloccocus aureus* to collect antigen-antibody complexes as described by Martial, J. A. et al., *Proc. Nat. Acad. Sci. USA 74*, 1816 (1977). The proteins were solubilized and fractionated by electrophoresis in sodium dodecylsulfate-polyacrylamide gels. Results are shown in Figure 4.

Lane (a) contains $^{14}$C-labeled proteins from bacteriophage T4-infected *E. coli* cells, used as size markers. Tracks (b) through (g) show pulse-labeled proteins at 0, 0.5, 1, 2, 3 and 4 hours, respectively, after induction. Track (h) shows the immunoprecipitated proteins from 4 hour induced cultures, with antiserum against human growth hormone. Track (i) shows the immunoprecipitate of the 4 hour induced pulse-labeled proteins with non-immune serum. Track (j) shows the immunoprecipitate of pulse-labeled, uninduced proteins. The upper and lower arrows show, respectively, the locations of the *trp*E and expected *trp*D-HGH fusion proteins. Numbers on the left indicate molecular weights x $10^{-3}$ of the marker proteins.

The results show the appearance of the *trp*D-HGH protein following induction and its specific precipitation by anti-human growth hormone serum. There is also a certain amount of immunoprecipitated *trp*E protein. Since the *trp*E and *trp*D proteins are normally associated in a complex, it seems likely that some association occurs between the *trp*E protein and the *trp*D-HGH fusion protein, by virtue of the *trp*D portion of the fusion protein. In any case, the evidence demonstrates the expression of the cloned human pre-growth hormone gene in *E. coli*.

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth, and as follows in the scope of the appended claims.

**Claims** for the contracting states: BE, CH, DE, FR, GB, IT, LI, LU, NL, SE

1. A DNA transfer vector comprising a deoxynucleotide sequence coding for human pre-growth hormone comprising:

$5'$-GGATCCTGTGGACAGCTCACCTAGCTGCAA $TG_{-26}$ $GCL_{-25}$ $ACL_{-24}$ $GGL_{-23}$ $QR_{-22}$ $S_{-22}$ $W_{-21}$ $GZ_{-21}$ $ACL_{-20}$ $QR_{-19}$ $S_{-19}$ $X_{-18}$ $TY_{-18}$ $X_{-17}$ $TX_{-17}$ $X_{-16}$ $TY_{-16}$ $GCL_{-15}$ $TTK_{-14}$ $GGL_{-13}$ $X_{-12}$ $TY_{-12}$ $X_{-11}$ $TY_{-11}$ $TGK_{-10}$ $X_{-9}$ $TY_{-9}$ $CCL_{-8}$ $TGG_{-7}$ $X_{-6}$ . $CAJ_{-5}$ $GAJ_{-4}$ $GCL_{-3}$ $GTL_{-2}$ $CCL_{-1}$ $TTK_1$ $CCL_2$ $ACL_3$ $ATM_4$ $CCL_5$ $X_6$ $TY_6$ $QR_7$ $S_7$ $W_8$ $GZ_8$ $X_9$ $TY_9$ $TTK_{10}$ $GAK_{11}$ $AAK_{12}$ $GCL_{13}$ $ATG_{14}$ $X_{15}$ $TY_{15}$ $W_{16}$ $GZ_{16}$ $GCL_{17}$ $CAK_{18}$ $W_{19}$ $GZ_{19}$ $X_{20}$ $TY_{20}$ $CAK_{21}$ $CAJ_{22}$ $X_{23}$ $TY_{23}$ $GCL_{24}$ $TTK_{25}$ $GAK_{26}$ $ACL_{27}$ $TAK_{28}$ $CAJ_{29}$ $GAJ_{30}$ $TTK_{31}$ $GAJ_{32}$ $GAJ_{33}$ $ACL_{34}$ $TAK_{35}$ $ATM_{36}$ $CCL_{37}$ $AAJ_{38}$ $GAJ_{39}$ $CAJ_{40}$ $AAJ_{41}$ $TAK_{42}$ $QR_{43}$ $S_{43}$ $TTK_{44}$ $X_{45}$ $TY_{45}$ $CAJ_{46}$ $AAK_{47}$ $CCL_{48}$ $CAJ_{49}$ $ACL_{50}$ $QR_{51}$ $S_{51}$ $X_{52}$ $TY_{52}$ $TGK_{53}$ $TTK_{54}$ $QR_{55}$ $S_{55}$ $GAJ_{56}$ $QR_{57}$ $S_{57}$ $ATM_{58}$ $CCL_{59}$ $ACL_{60}$ $CCL_{61}$ $QR_{62}$ $S_{62}$ $AAK_{63}$ $W_{64}$ $GZ_{64}$ $GAJ_{65}$ $GAJ_{66}$ $ACL_{67}$ $CAJ_{68}$ $CAJ_{69}$ $AAJ_{70}$ $QR_{71}$ $S_{71}$ $AAK_{72}$ $X_{73}$ $TY_{73}$

$GAJ_{74}$ $X_{75}$ $TY_{75}$ $X_{76}$ $TY_{76}$ $W_{77}$ $GZ_{77}$ $ATM_{78}$ $QR_{79}$ $S_{79}$ $X_{80}$ $TY_{80}$ $X_{81}$ $TY_{81}$ $X_{82}$ $TY_{82}$ $ATM_{83}$ $CAJ_{84}$ $QR_{85}$ $S_{85}$ $TGGX_{87}$ $TY_{87}$ $GAJ_{88}$ $CCL_{89}$ $GTL_{90}$ $CAJ_{91}$ $TTK_{92}$ $X_{93}$ $TY_{93}$ $W_{94}$ $GZ_{94}$ $QR_{95}$ $S_{95}$ $GTL_{96}$ $TTK_{97}$ $GCL_{98}$ $AAK_{99}$ $AAK_{100}$ $X_{101}$ $TY_{101}$ $GTL_{102}$ $TAK_{103}$ $GGL_{104}$ $GCL_{105}$ $QR_{106}$ $S_{106}$ $GAK_{107}$ $QR_{108}$ $S_{108}$ $AAK_{109}$ $GTL_{110}$ $TAK_{111}$ $GAK_{112}$ $X_{113}$ $TY_{113}$ $X_{114}$ $TY_{114}$ $AAJ_{115}$ $GAK_{116}$ $X_{117}$ $TY_{117}$ $GAJ_{118}$ $GAJ_{119}$ $GGL_{120}$ $ATM_{121}$ $CAJ_{122}$ $ACL_{123}$ $X_{124}$ $TY_{124}$ $ATGGGL_{126}$ $W_{127}$ $GZ_{127}$ $X_{128}$ $TY_{128}$ $GAJ_{129}$ $GAK_{130}$ $GGL_{131}$ $QR_{132}$ $S_{132}$ $CCL_{133}$ $W_{134}$ $GZ_{134}$ $ACL_{135}$ $GGL_{136}$ $CAJ_{137}$ $ATM_{138}$ $TTK_{139}$ $AAJ_{140}$ $CAJ_{141}$ $ACL_{142}$ $TAK_{143}$ $QR_{144}$ $S_{144}$ $AAJ_{145}$ $TTK_{146}$ $GAK_{147}$ $ACL_{148}$ $AAK_{149}$ $QR_{150}$ $S_{150}$ $CAK_{151}$ $AAK_{152}$ $CAK_{153}$ $GAK_{154}$ $GCL_{155}$ $X_{156}$ $TY_{156}$ $X_{157}$ $TY_{157}$ $AAJ_{158}$ $AAK_{159}$ $TAK_{160}$ $GGL_{161}$ $X_{162}$ $TY_{162}$ $X_{163}$ $TY_{163}$ $TAK_{164}$ $TGK_{165}$ $TTK_{166}$ $W_{167}$ $GZ_{167}$ $AAJ_{168}$ $GAK_{169}$ $ATGGAK_{171}$ $AAJ_{172}$ $GTL_{173}$ $GAJ_{174}$ $ACL_{175}$ $TTK_{176}$ $X_{177}$ $TY_{177}$ $W_{178}$ $GZ_{178}$ $ATM_{179}$ $GTL_{180}$ $CAJ_{181}$ $TGK_{182}$ $W_{183}$ $GZ_{183}$ $QR_{184}$ $S_{184}$ $GTL_{185}$ $GAJ_{186}$ $GGL_{187}$ $QR_{188}$ $S_{188}$ $TGK_{189}$ $GGL_{190}$ $TTK_{191}$ TAGCTGCCCGGGTGGCATCCCTGTGACCCCTCCCCAGTGCCT CTCCTGGCCCTGGAAGTTGCCACTCCAGTGCCCACCAGCCTTGTCCTAATAAAATTAAGT TGCATCpolyA-3'

wherein

A is deoxyadenyl;

G is deoxyguanyl;

C is deoxycytosyl;

T is thymidyl;

J is A or G;

K is T or C;

L is A, TC or G;

M is A, C or T;

$X_n$ is T or C, if $Y_n$ is A or G, and C if $Y_n$ is C or T;

$Y_n$ is A, G, C or T, if $X_n$ is C, and A or G if $X_n$ is T;

$W_n$ is C or A, if $Z_n$ is G or A, and C if $Z_n$ is C or T;

$Z_n$ is A, G, C or T, if $W_n$ is C and A or G if $W_n$ is A;

$QR_n$ is TC if $S_n$ is A, G, C or T, and AG if S is T or C;

$S_n$ is A, G, C or T if $QR_n$ is TC, and T or C if $QR_n$ is AG;

and subscript numerals, n, refer to the position in the amino acid sequence of human growth hormone, to which each triplet in the nucleotide sequence corresponds, according to the genetic code, the amino acid positions being numbered from the amino end.

2. A microorganism transformed by the transfer vector of claim 1 and capable of expressing human pre-growth hormone.

3. The plasmid pBR322 containing at the HindlII site thereof a DNA sequence as recited in claim 1.

4. A microorganism transformed by the plasmid of claim 3 and capable of expressing human pre-growth hormone.

5. The plasmid ptrpED50 containing at the HindlII site thereof a DNA sequence as recited om Claim 1.

6. A microorganism transformed by the plasmid of claim 5 and capable of expressing human pre-growth hormone.

7. Escherichia coli transformed by the plasmid of claim 5 and capable of expressing human pre-growth hormone.

8. A chimeric protein comprising the amino acid sequence of human pre-growth hormone as its C-terminal sequence, and a portion of a procaryotic protein as its N-terminal sequence, as expressed by a microorganism according to claim 6 or claim 7.

9. The protein of claim 6, wherein the procaryotic portion is coded by part of the trpD gene of Escherichia coli.

10. A method for cloning a deoxynucleotide sequence as recited in claim 1, coding for human pre-growth hormone, from pituitary gland tissue obtained by surgery of an individual human, comprising the steps of

a) extracting mRNA coding for human pre-growth hormone from an individual human pituitary gland,

b) separating the mRNA substantially free of protein, DNA and other RNA,

c) synthesizing a single-stranded cDNA having a nucleotide sequence complementary to that of the mRNA, in a series of procedures including incubation with an enzyme, extraction to remove protein from the reaction mixture, alcohol precipitation, and alkaline hydrolysis, all of said procedures being carried out in the same reaction tube, in the absence of added carrier DNA,

d) purifying the single-stranded cDNA by chromatography wherein those fractions containing the single-stranded DNA are located by a radioassay which does not consume the reaction product,

e) synthesizing double-stranded cDNA having one strand with a nucleotide sequence corresponding to that of the mRNA, in a series of procedures including incubation with an enzyme and extraction to remove protein, said procedures being carried out in a single reaction tube in the absence of added carrier DNA.

f) purifying the double-stranded cDNA by chromatography as in step d).

g) further treating the double-stranded cDNA with enzyme, essentially as described in steps e) and f) to provide end groups suitable for inserting the cDNA into a DNA transfer vector,

h) inserting the double-stranded cDNA into a DNA transfer vector, thereby forming a recombinant transfer vector,

i) transforming a microorganism with the recombinant transfer vector, and

j) selecting a microorganism strain derived from a single transformed cell transformed by a recombinant transfer vector containing cDNA coding for human pregrowth hormone, thereby cloning a deoxynucleotide sequence coding for human pre-growth hormone.

**Claims** for the contracting state: AT

1. A process for modifying a DNA transfer vector comprising inserting in the DNA sequence of said transfer vector a fragment of heterologous DNA, having the following sequence:

5'-GGATCCTGTGGACAGCTCACCTAGCTGCAA $TG_{-26}$ $GCL_{-25}$ $ACL_{-24}$ $GGL_{-23}$ $QR_{-22}$ $S_{-22}$ $W_{-21}$ $GZ_{-21}$ $ACL_{-20}$ $QR_{-19}$ $S_{-19}$ $X_{-18}$ $TY_{-18}$ $X_{-17}$ $TX_{-17}$ $X_{-16}$ $TY_{-16}$ $GCL_{-15}$ $TTK_{-14}$ $GGL_{-13}$ $X_{-12}$ $TY_{-12}$ $X_{-11}$ $TY_{-11}$ $TGK_{-10}$ $X_{-9}$ $TY_{-9}$ $CCL_{-8}$ $TGG_{-7}$ $X_{-6}$ $CAJ_{-5}$ $GAJ_{-4}$ $GCL_{-3}$ $GTL_{-2}$ $CCL_{-1}$ $TTK_1$ $CCL_2$ $ACL_3$ $ATM_4$ $CCL_5$ $X_6$ $TY_6$ $QR_7$ $S_7$ $W_8$ $GZ_8$ $X_9$ $TY_9$ $TTK_{10}$ $GAK_{11}$ $AAK_{12}$ $GCL_{13}$ $ATG_{14}$ $X_{15}$ $TY_{15}$ $W_{16}$ $GZ_{16}$ $GCL_{17}$ $CAK_{18}$ $W_{19}$ $GZ_{19}$ $X_{20}$ $TY_{20}$ $CAK_{21}$ $CAJ_{22}$ $X_{23}$ $TY_{23}$ $GCL_{24}$ $TTK_{25}$ $GAK_{26}$ $ACL_{27}$ $TAK_{28}$ $CAJ_{29}$ $GAJ_{30}$ $TTK_{31}$ $GAJ_{32}$ $GAJ_{33}$ $ACL_{34}$ $TAK_{35}$ $ATM_{36}$ $CCL_{37}$ $AAJ_{38}$ $GAJ_{39}$ $CAJ_{40}$ $AAJ_{41}$ $TAK_{42}$ $QR_{43}$ $S_{43}$ $TTK_{44}$ $X_{45}$ $TY_{45}$ $CAJ_{46}$ $AAK_{47}$ $CCL_{48}$ $CAJ_{49}$ $ACL_{50}$ $QR_{51}$ $S_{51}$ $X_{52}$ $TY_{52}$ $TGK_{53}$ $TTK_{54}$ $QR_{55}$ $S_{55}$ $GAJ_{56}$ $QR_{57}$ $S_{57}$ $ATM_{58}$ $CCL_{59}$ $ACL_{60}$ $CCL_{61}$ $QR_{62}$ $S_{62}$ $AAK_{63}$ $W_{64}$ $GZ_{64}$ $GAJ_{65}$ $GAJ_{66}$ $ACL_{67}$ $CAJ_{68}$ $CAJ_{69}$ $AAJ_{70}$ $QR_{71}$ $S_{71}$ $AAK_{72}$ $X_{73}$ $TY_{73}$ $GAJ_{74}$ $X_{75}$ $TY_{75}$ $X_{76}$ $TY_{76}$ $W_{77}$ $GZ_{77}$ $ATM_{78}$ $QR_{79}$ $S_{79}$ $X_{80}$ $TY_{80}$ $X_{81}$ $TY_{81}$ $X_{82}$ $TY_{82}$ $ATM_{83}$ $CAJ_{84}$ $QR_{85}$ $S_{85}$ $TGGX_{87}$ $TY_{87}$ $GAJ_{88}$ $CCL_{89}$ $GTL_{90}$ $CAJ_{91}$ $TTK_{92}$ $X_{93}$ $TY_{93}$ $W_{94}$ $GZ_{94}$ $QR_{95}$ $S_{95}$ $GTL_{96}$ $TTK_{97}$ $GCL_{98}$ $AAK_{99}$ $AAK_{100}$ $X_{101}$ $TY_{101}$ $GTL_{102}$ $TAK_{103}$ $GGL_{104}$ $GCL_{105}$ $QR_{106}$ $S_{106}$ $GAK_{107}$ $QR_{108}$ $S_{108}$ $AAK_{109}$ $GTL_{110}$ $TAK_{111}$ $GAK_{112}$ $X_{113}$ $TY_{113}$ $X_{114}$ $TY_{114}$ $AAJ_{115}$ $GAK_{116}$ $X_{117}$ $TY_{117}$ $GAJ_{118}$ $GAJ_{119}$ $GGL_{120}$ $ATM_{121}$ $CAJ_{122}$ $ACL_{123}$ $X_{124}$ $TY_{124}$ $ATGGGL_{126}$ $W_{127}$ $GZ_{127}$ $X_{128}$ $TY_{128}$ $GAJ_{129}$ $GAK_{130}$ $GGL_{131}$ $QR_{132}$ $S_{132}$ $CCL_{133}$ $W_{134}$ $GZ_{134}$ $ACL_{135}$ $GGL_{136}$ $CAJ_{137}$ $ATM_{138}$ $TTK_{139}$ $AAJ_{140}$ $CAJ_{141}$ $ACL_{142}$ $TAK_{143}$ $QR_{144}$ $S_{144}$ $AAJ_{145}$ $TTK_{146}$ $GAK_{147}$ $ACL_{148}$ $AAK_{149}$ $QR_{150}$ $S_{150}$ $CAK_{151}$ $AAK_{152}$ $CAK_{153}$ $GAK_{154}$ $GCL_{155}$ $X_{156}$ $TY_{156}$ $X_{157}$ $TY_{157}$ $AAJ_{158}$ $AAK_{159}$ $TAK_{160}$ $GGL_{161}$ $X_{162}$ $TY_{162}$ $X_{163}$ $TY_{163}$ $TAK_{164}$ $TGK_{165}$ $TTK_{166}$ $W_{167}$ $GZ_{167}$ $AAJ_{168}$ $GAK_{169}$ $ATGGAK_{171}$ $AAJ_{172}$ $GTL_{173}$ $GAJ_{174}$ $ACL_{175}$ $TTK_{176}$ $X_{177}$ $TY_{177}$ $W_{178}$ $GZ_{178}$ $ATM_{179}$ $GTL_{180}$ $CAJ_{181}$ $TGK_{182}$ $W_{183}$ $GZ_{183}$ $QR_{184}$ $S_{184}$ $GTL_{185}$ $GAJ_{186}$ $GGL_{187}$ $QR_{188}$ $S_{188}$ $TGK_{189}$ $GGL_{190}$ $TTK_{191}$ TAGCTGCCCGGGTGGCATCCCTGTGACCCCTCCCCAGTGCCT CTCCTGGCCCTGGAAGTTGCCACTCCAGTGCCCACCAGCCTTGTCCTAATAAAATTAAGT TGCATCpolyA-3'

wherein
A is deoxyadenyl;
G is deoxyguanyl;
C is deoxycytosyl;
T is thymidyl;
J is A or G;
K is T or C;
L is A, TC or G;
M is A, C or T;
$X_n$ is T or C, if $Y_n$ is A or G, and C if $Y_n$ is C or T;
$Y_n$ is A; G, C or T, if $X_n$ is C, and A or G if $X_n$ is T;
$W_n$ is C or A, if $Z_n$ is G or A, and C if $Z_n$ is C or T;
$Z_n$ is A, G, C or T, if $W_n$ is C and A or G if $W_n$ is A;
$QR_n$ is TC if $S_n$ is A, G, C or T, and AG if S is T or C;
$S_n$ is A, G, C or T if $QR_n$ is TC, and T or C if $QR_n$ is AG;
and subscript numerals, n, refer to the position in the amino acid sequence of human growth hormone, to which each triplet in the nucleotide sequence corresponds, according to the genetic code, the amino acid positions being numbered from the amino end.

2. A process according to claim 1, wherein the fragment of heterologous DNA is inserted at the *Hind*III site of the plasmid pBR322.

3. A process according to claim 1, wherein the fragment of heterologous DNA is inserted at the *Hind*III site of the plasmid p*trp*ED-50.

4. A process for producing a microorganism capable of expressing human pre-growth hormone, comprising transforming said microorganism with a transfer vector obtained by the process of any preceding claim.

5. A process according to claim 4, wherein the microorganism is *Escherichia coli.*

6. A process for synthesising a chimeric protein comprising the amino acid sequence of human pre-growth hormone as its C-terminal sequence, and a portion of a procaryotic protein as its N-terminal sequence, said process comprising incubating a microorganism obtained by the process of claim 4 or claim 5.

7. The process of claim 6, wherein the procaryotic portion is coded by part of the *trp*D gene of *Escherichia coli.*

8. A method for cloning a deoxynucleotide sequence as recited in claim 1, coding for coding for human pregrowth hormone, from pituitary gland tissue obtained by surgery of an individual human, comprising the steps of

a) extracting mRNA coding for human pre-growth hormone from an individual human pituitary gland,

b) separating the mRNA substantially free of protein, DNA and other RNA,

c) synthesizing a single-stranded cDNA having a nucleotide sequence complementary to that of the mRNA, in a series of procedures including incubation with an enzyme, extraction to remove protein from the reaction mixture, alcohol precipitation, and alkaline hydrolysis, all of said procedures being carried out in the same reaction tube, in the absence of added carrier DNA,

d) purifying the single-stranded cDNA by chromatography wherein those fractions containing the single-stranded DNA are located by a radioassay which does not consume the reaction product,

e) synthesizing double-stranded cDNA having one strand with a nucleotide sequence corresponding to that of the mRNA, in a series of procedures including incubation with an enzyme, and extraction to remove protein, said procedures being carried out in a single reaction tube in the absence of added carrier DNA.

f) purifying the double-stranded cDNA by chromatography as in step d),

g) further treating the double-stranded cDNA with enzyme essentially as described in steps e) and f) to provide end groups suitable for inserting the cDNA into a DNA transfer vector,

h) inserting the double-stranded cDNA into a DNA transfer vector, thereby forming a recombinant transfer vector,

i) transforming a microorganism with the recombinant transfer vector, and

j) selecting a microorganism strain derived from a single transformed cell transformed by a recombinant transfer vector containing cDNA coding for human pregrowth hormone, thereby cloning a deoxynucleotide sequence coding for human pre-growth hormone.

**Patentansprüche** für die Vertragsstaaten: BE, CH, DE, FR, GB, IT, LI, LU, NL, SE

1. Ein DNA-Übertragungsvektor, enthaltend eine Desoxynukleotidsequenz, welche für menschlichen Wachstumshormonvorläufer kodiert und umfaßt:

5'-GGATCCTGTGGACAGCTCACCTAGCTGCAA $TG_{-26}$ $GCL_{-25}$ $ACL_{-24}$ $GGL_{-23}$ $QR_{-22}$ $S_{-22}$ $W_{-21}$ $GZ_{-21}$ $ACL_{-20}$ $QR_{-19}$ $S_{-19}$ $X_{-18}$ $TY_{-18}$ $X_{-17}$ $TX_{-17}$ $X_{-16}$ $TY_{-16}$ $GCL_{-15}$ $TTK_{-14}$ $GGL_{-13}$ $X_{-12}$ $TY_{-12}$ $X_{-11}$ $TY_{-11}$ $TGK_{-10}$ $X_{-9}$ $TY_{-9}$ $CCL_{-8}$ $TGG_{-7}$ $X_{-6}$ $CAJ_{-5}$ $GAJ_{-4}$ $GCL_{-3}$ $GTL_{-2}$ $CCL_{-1}$ $TTK_1$ $CCL_2$ $ACL_3$ $ATM_4$ $CCL_5$ $X_6$ $TY_6$ $QR_7$ $S_7$ $W_8$ $GZ_8$ $X_9$ $TY_9$ $TTK_{10}$ $GAK_{11}$ $AAK_{12}$ $GCL_{13}$ $ATG_{14}$ $X_{15}$ $TY_{15}$ $W_{16}$ $GZ_{16}$ $GCL_{17}$ $CAK_{18}$ $W_{19}$ $GZ_{19}$ $X_{20}$ $TY_{20}$ $CAK_{21}$ $CAJ_{22}$ $X_{23}$ $TY_{23}$ $GCL_{24}$ $TTK_{25}$ $GAK_{26}$ $ACL_{27}$ $TAK_{28}$ $CAJ_{29}$ $GAJ_{30}$ $TTK_{31}$ $GAJ_{32}$ $GAJ_{33}$ $ACL_{34}$ $TAK_{35}$ $ATM_{36}$ $CCL_{37}$ $AAJ_{38}$ $GAJ_{39}$ $CAJ_{40}$ $AAJ_{41}$ $TAK_{42}$ $QR_{43}$ $S_{43}$ $TTK_{44}$ $X_{45}$ $TY_{45}$ $CAJ_{46}$ $AAK_{47}$ $CCL_{48}$ $CAJ_{49}$ $ACL_{50}$ $QR_{51}$ $S_{51}$ $X_{52}$ $TY_{52}$ $TGK_{53}$ $TTK_{54}$ $QR_{55}$ $S_{55}$ $GAJ_{56}$ $QR_{57}$ $S_{57}$ $ATM_{58}$ $CCL_{59}$ $ACL_{60}$ $CCL_{61}$ $QR_{62}$ $S_{62}$ $AAK_{63}$ $W_{64}$ $GZ_{64}$ $GAJ_{65}$ $GAJ_{66}$ $ACL_{67}$ $CAJ_{68}$ $CAJ_{69}$ $AAJ_{70}$ $QR_{71}$ $S_{71}$ $AAK_{72}$ $X_{73}$ $TY_{73}$ $GAJ_{74}$ $X_{75}$ $TY_{75}$ $X_{76}$ $TY_{76}$ $W_{77}$ $GZ_{77}$ $ATM_{78}$ $QR_{79}$ $S_{79}$ $X_{80}$ $TY_{80}$ $X_{81}$ $TY_{81}$ $X_{82}$ $TY_{82}$ $ATM_{83}$ $CAJ_{84}$ $QR_{85}$ $S_{85}$ $TGGX_{87}$ $TY_{87}$ $GAJ_{88}$ $CCL_{89}$ $GTL_{90}$ $CAJ_{91}$ $TTK_{92}$ $X_{93}$ $TY_{93}$ $W_{94}$ $GZ_{94}$ $QR_{95}$ $S_{95}$ $GTL_{96}$ $TTK_{97}$ $GCL_{98}$ $AAK_{99}$ $AAK_{100}$ $X_{101}$ $TY_{101}$ $GTL_{102}$ $TAK_{103}$ $GGL_{104}$ $GCL_{105}$ $QR_{106}$ $S_{106}$ $GAK_{107}$ $QR_{108}$ $S_{108}$ $AAK_{109}$ $GTL_{110}$ $TAK_{111}$ $GAK_{112}$ $X_{113}$ $TY_{113}$ $X_{114}$ $TY_{114}$ $AAJ_{115}$ $GAK_{116}$ $X_{117}$ $TY_{117}$ $GAJ_{118}$ $GAJ_{119}$ $GGL_{120}$ $ATM_{121}$ $CAJ_{122}$ $ACL_{123}$ $X_{124}$ $TY_{124}$ $ATGGGL_{126}$ $W_{127}$ $GZ_{127}$ $X_{128}$ $TY_{128}$ $GAJ_{129}$ $GAK_{130}$ $GGL_{131}$ $QR_{132}$ $S_{132}$ $CCL_{133}$ $W_{134}$ $GZ_{134}$ $ACL_{135}$ $GGL_{136}$ $CAJ_{137}$ $ATM_{138}$ $TTK_{139}$ $AAJ_{140}$ $CAJ_{141}$ $ACL_{142}$ $TAK_{143}$ $QR_{144}$ $S_{144}$ $AAJ_{145}$ $TTK_{146}$ $GAK_{147}$ $ACL_{148}$ $AAK_{149}$ $QR_{150}$ $S_{150}$ $CAK_{151}$ $AAK_{152}$ $CAK_{153}$ $GAK_{154}$ $GCL_{155}$ $X_{156}$ $TY_{156}$ $X_{157}$ $TY_{157}$ $AAJ_{158}$ $AAK_{159}$ $TAK_{160}$ $GGL_{161}$ $X_{162}$ $TY_{162}$ $X_{163}$ $TY_{163}$ $TAK_{164}$ $TGK_{165}$ $TTK_{166}$ $W_{167}$ $GZ_{167}$ $AAJ_{168}$ $GAK_{169}$ $ATGGAK_{171}$ $AAJ_{172}$ $GTL_{173}$ $GAJ_{174}$ $ACL_{175}$ $TTK_{176}$ $X_{177}$ $TY_{177}$ $W_{178}$ $GZ_{178}$ $ATM_{179}$ $GTL_{180}$ $CAJ_{181}$ $TGK_{182}$ $W_{183}$ $GZ_{183}$ $QR_{184}$ $S_{184}$ $GTL_{185}$ $GAJ_{186}$ $GGL_{187}$ $QR_{188}$ $S_{188}$ $TGK_{189}$ $GGL_{190}$ $TTK_{191}$ TAGCTGCCCGGGTGGCATCCCTGTGACCCCTCCCCAGTGCCT CTCCTGGCCCTGGAAGTTGCCACTCCAGTGCCCACCAGCCTTGTCCTAATAAAATTAAGT TGCATCpolyA-3'

worin A = Desoxyadenyl, G = Desoxyguanyl, C = Desoxycytosyl, T = Thymidyl, J = A oder G; K = T oder C, L = A, TC oder G, M = A, C oder T, $X_n$ = T oder C, wenn $Y_n$ = A oder G, und C, wenn $Y_n$ = C oder T; $Y_n$ = A, G, C oder T, wenn $X_n$ = C, und A oder G, wenn $X_n$ = T; $W_n$ = C oder A, wenn $Z_n$ = G oder A, und C, wenn $Z_n$ = C oder T; $Z_n$ = A, G, C oder T, wenn $W_n$ = C, und A oder G, wenn $W_n$ = A; $QR_n$ = TC, wenn $S_n$ = A, G, C oder T, und AG, wenn $S_n$ = T oder C; $S_n$ = A, G, C oder T, wenn $QR_n$ = TC, und T oder C, wenn $QR_n$ = AG, wobei die tiefgestellten Zahlen n sich auf die Stelle, der jedes Triplett in der Nukleotidsequenz nach dem genetischen Kode entspricht, in der Aminosäuresequenz des menschlichen Wachstumshormons beziehen, und wobei die Aminosäurestellen von Aminende her durchnumeriert sind.

2. Ein Mikroorganismus, der durch den Übertragungsvektor nach Anspruch 1 transformiert wurde und zur Expression von menschlichem Wachstumshormonvorläufer fähig ist.

3. Das Plasmid pBR322, enthaltend an der *Hind*III-Stelle, hievon eine DNA Sequenz gemäß Anspruch 1.

4. Ein Mikroorganismus, der durch das Plasmid von Anspruch 3 transformiert wurde und zur Expression von menschlichem Wachstumshormonvorläufer fähig ist.

5. Das Plasmid p*trp*ED5O, enthaltend an seiner *Hind*III Stelle eine DNA Sequenz gemäß Anspruch 1.

6. Ein Mikroorganismus, transforiert durch das Plasmid nach Anspruch 5 und fähig zur Expression von menschlichem Wachstumshormonvorläufer.

7. *Escherichia coli*, transformiert durch das Plasmid nach Anspruch 5 und fähig zur Expression von menschlichem Wachstumshormonvorläufer.

8. Ein chimäres Protein, bestehend aus einer Aminosäuresequenz des menschlichen Wachstumshormonvorläufers als C-terminale Sequenz, und einem Teil eines prokaryotischen Protein als N-terminale Sequenz, wie ausgedrückt durch einen Mikroorganismus nach Anspruch 6 oder 7.

9. Protein nach Anspruch 8, worin der prokaryotische Teil von einem Teil des *trp*D-Gens von *Escherichia coli* kodiert ist.

10. Methode zum Klonieren einer Desoxynukleotidsequenz nach Anspruch 1, welche für menschlichen Wachstumshormonvorläufer kodiert aus Hypophysengewebe, erhalten auf chirurgischem Wege aus einem einzigen Menschen, umfassend die folgenden Stufen:

a) Extraktion von für menschlichen Wachstumshormonvorläufer kodierender mRNA aus einer einzigen menschlichen Hypophyse,

b) Abtrennung im, wesentlichen von Protein, DNA und anderer RNA freier mRNA,

c) Synthese einer einsträngigen cDNA mit einer der Nukleotidsequenz der mRNA komplementären Sequenz durch eine Reihe von Verfahren, darunter Inkubation mit einem Enzym, Extraktion zwecks Abtrennen von Proteinen aus dem Reaktionsgemisch, Äthanolfällung und basische Hydrolyse, wobei sämtliche besagte Verfahren in demselben Reaktionsröhrchen in Abwesenheit zugesetzter Träger-DNA durchgeführt werden,

d) chromatographische Reinigung der einsträngigen cDNA, wobei die die einsträngige DNA enthaltend Fraktionen mittels einer das Reaktionsprodukt nicht verbrauchenden Radiobestimmungsmethode nachgewiesen werden,

e) Synthese der doppelsträngigen cDNa mit einem Strang, dessen Nukleotidsequenz der mRNA entspricht, durch eine Reihe von Verfahren, darunter Inkubation mit einem Enzym und Extraktion zwecks Abtrennen von Proteinen, wobei besagte Verfahren in einem einzigen Reaktionsröhrchen Abwesenheit zugesetzter Träger-DNA durchgeführt werden,

f) chromatographische Reinigung der doppelsträngigen cDNA wie bei Stufe d),

g) weitere Behandlung der doppelsträngigen cDNA mit Enzym, im wesentlichen wie bei Stufen e) und f) beschrieben, um geeignete Endgruppen bereitzustellen,

h) Insertion der doppelsträngigen cDNA in einen DNA-Übertragungsvektor, wobei ein rekombinanter Übertragungsvektor gebildet wird,

i) Transformation eines Mikroorganismus mit einem rekombinanten Übertragungsvektor, und

j) Selektion eines Mikroorganismusstamms, der von einer einzigen von einem rekombinanten Übertragungsvektor mit für menschlichen Wachstumshormonvorläufer kodierender cDNA transformierten Zelle abstammt, wobei eine für menschlichen Wachstumshormonvorläufer kodierende Desoxynukleotidsequenz kloniert wird.

**Patentansprüche** für den Vertragstaat: AT

1. Verfahren zur Abänderung eines DNA-Übertragungsvektors, bestehend aus der Insertion eines Bruchstückes heterologer DNA in die DNA Sequenz des besagten Übertragungsvektors mit folgender Sequenz:

5'-GGATCCTGTGGACAGCTCACCTAGCTGCAA $TG_{-26}$ $GCL_{-25}$ $ACL_{-24}$ $GGL_{-23}$ $QR_{-22}$ $S_{-22}$ $W_{-21}$ $GZ_{-21}$ $ACL_{-20}$ $QR_{-19}$ $S_{-19}$ $X_{-18}$ $TY_{-18}$ $X_{-17}$ $TX_{-17}$ $X_{-16}$ $TY_{-16}$ $GCL_{-15}$ $TTK_{-14}$ $GGL_{-13}$ $X_{-12}$ $TY_{-12}$ $X_{-11}$ $TY_{-11}$ $TGK_{-10}$ $X_{-9}$ $TY_{-9}$ $CCL_{-8}$ $TGG_{-7}$ $X_{-6}$ $CAJ_{-5}$ $GAJ_{-4}$ $GCL_{-3}$ $GTL_{-2}$ $CCL_{-1}$ $TTK_{1}$ $CCL_{2}$ $ACL_{3}$ $ATM_{4}$ $CCL_{5}$ $X_{6}$ $TY_{6}$ $QR_{7}$ $S_{7}$ $W_{8}$ $GZ_{8}$ $X_{9}$ $TY_{9}$ $TTK_{10}$ $GAK_{11}$ $AAK_{12}$ $GCL_{13}$ $ATG_{14}$ $X_{15}$ $TY_{15}$ $W_{16}$ $GZ_{16}$ $GCL_{17}$ $CAK_{18}$ $W_{19}$ $GZ_{19}$ $X_{20}$ $TY_{20}$ $CAK_{21}$ $CAJ_{22}$ $X_{23}$ $TY_{23}$ $GCL_{24}$ $TTK_{25}$ $GAK_{26}$ $ACL_{27}$ $TAK_{28}$ $CAJ_{29}$ $GAJ_{30}$ $TTK_{31}$ $GAJ_{32}$ $GAJ_{33}$ $ACL_{34}$ $TAK_{35}$ $ATM_{36}$ $CCL_{37}$ $AAJ_{38}$ $GAJ_{39}$ $CAJ_{40}$ $AAJ_{41}$ $TAK_{42}$ $QR_{43}$ $S_{43}$ $TTK_{44}$ $X_{45}$ $TY_{45}$ $CAJ_{46}$ $AAK_{47}$ $CCL_{48}$ $CAJ_{49}$ $ACL_{50}$ $QR_{51}$ $S_{51}$ $X_{52}$ $TY_{52}$ $TGK_{53}$ $TTK_{54}$ $QR_{55}$ $S_{55}$ $GAJ_{56}$ $QR_{57}$ $S_{57}$ $ATM_{58}$ $CCL_{59}$ $ACL_{60}$ $CCL_{61}$ $QR_{62}$ $S_{62}$ $AAK_{63}$ $W_{64}$ $GZ_{64}$ $GAJ_{65}$ $GAJ_{66}$ $ACL_{67}$ $CAJ_{68}$ $CAJ_{69}$ $AAJ_{70}$ $QR_{71}$ $S_{71}$ $AAK_{72}$ $X_{73}$ $TY_{73}$ $GAJ_{74}$ $X_{75}$ $TY_{75}$ $X_{76}$ $TY_{76}$ $W_{77}$ $GZ_{77}$ $ATM_{78}$ $QR_{79}$ $S_{79}$ $X_{80}$ $TY_{80}$ $X_{81}$ $TY_{81}$ $X_{82}$ $TY_{82}$ $ATM_{83}$ $CAJ_{84}$ $QR_{85}$ $S_{85}$ $TGGX_{87}$ $TY_{87}$ $GAJ_{88}$ $CCL_{89}$ $GTL_{90}$ $CAJ_{91}$ $TTK_{92}$ $X_{93}$ $TY_{93}$ $W_{94}$ $GZ_{94}$ $QR_{95}$ $S_{95}$ $GTL_{96}$ $TTK_{97}$ $GCL_{98}$ $AAK_{99}$ $AAK_{100}$ $X_{101}$ $TY_{101}$ $GTL_{102}$ $TAK_{103}$ $GGL_{104}$ $GCL_{105}$ $QR_{106}$ $S_{106}$ $GAK_{107}$ $QR_{108}$ $S_{108}$ $AAK_{109}$ $GTL_{110}$ $TAK_{111}$ $GAK_{112}$ $X_{113}$ $TY_{113}$ $X_{114}$ $TY_{114}$ $AAJ_{115}$ $GAK_{116}$ $X_{117}$ $TY_{117}$ $GAJ_{118}$ $GAJ_{119}$ $GGL_{120}$ $ATM_{121}$ $CAJ_{122}$ $ACL_{123}$ $X_{124}$ $TY_{124}$ $ATGGGL_{126}$ $W_{127}$ $GZ_{127}$ $X_{128}$ $TY_{128}$ $GAJ_{129}$ $GAK_{130}$ $GGL_{131}$ $QR_{132}$ $S_{132}$ $CCL_{133}$ $W_{134}$ $GZ_{134}$ $ACL_{135}$ $GGL_{136}$ $CAJ_{137}$ $ATM_{138}$ $TTK_{139}$ $AAJ_{140}$ $CAJ_{141}$ $ACL_{142}$ $TAK_{143}$ $QR_{144}$ $S_{144}$ $AAJ_{145}$ $TTK_{146}$ $GAK_{147}$ $ACL_{148}$ $AAK_{149}$ $QR_{150}$ $S_{150}$ $CAK_{151}$ $AAK_{152}$ $CAK_{153}$ $GAK_{154}$ $GCL_{155}$ $X_{156}$ $TY_{156}$ $X_{157}$ $TY_{157}$ $AAJ_{158}$ $AAK_{159}$ $TAK_{160}$ $GGL_{161}$ $X_{162}$ $TY_{162}$ $X_{163}$ $TY_{163}$ $TAK_{164}$ $TGK_{165}$ $TTK_{166}$ $W_{167}$ $GZ_{167}$ $AAJ_{168}$ $GAK_{169}$ $ATGGAK_{171}$ $AAJ_{172}$ $GTL_{173}$ $GAJ_{174}$ $ACL_{175}$ $TTK_{176}$ $X_{177}$ $TY_{177}$ $W_{178}$ $GZ_{178}$ $ATM_{179}$ $GTL_{180}$ $CAJ_{181}$ $TGK_{182}$ $W_{183}$ $GZ_{183}$ $QR_{184}$ $S_{184}$ $GTL_{185}$ $GAJ_{186}$ $GGL_{187}$ $QR_{188}$ $S_{188}$ $TGK_{189}$ $GGL_{190}$ $TTK_{191}$ TAGCTGCCCGGGTGGCATCCCTGTGACCCCTCCCCAGTGCCT CTCCTGGCCCTGGAAGTTGCCACTCCAGTGCCCACCAGCCTTGTCCTAATAAAATTAAGT TGCATCpolyA-3'

worin A=Desoxyadenyl, G=Desoxyguanyl, C=Desoxycytosyl, T=Thymidyl, J=A oder G; K=T oder C, L=A, TC oder G, M=A, C oder T, $X_n$=T oder C, wenn $Y_n$=A oder G, und C, wenn $Y_n$=C oder T; $Y_n$=A, G, C oder T, wenn $X_n$=C, und A oder G, wenn $X_n$=T; $W_n$=C oder A, wenn $Z_n$=G oder A, und C, wenn $Z_n$=C oder T; $Z_n$=A, G, C oder T, wenn $W_n$=C, und A oder G, wenn $W_n$=A; $QR_n$=TC, wenn $S_n$=A, G, C oder T, und AG, wenn $S_n$=T oder C; $S_n$=A, G, C oder T, wenn $QR_n$=TC, und T oder C, wenn $QR_n$=AG, wobei die tiefgestellten Zahlen n sich auf die Stelle, der jedes Triplett in der Nukleotidsequenz nach dem genetischen Kode entspricht, in der Aminosäuresequenz des menschlichen Wachstumshormons beziehen, und wobei die Aminosäurestellen von Aminende her durchnumeriert sind.

2. Verfahren nach Anspruch 1, dadurch gekennzeichnet, daß das Bruchstück der heterologen DNA an der HindIII-Stelle des Plasmids pBR 22 eingebaut wird.

3. Verfahren nach Anspruch 1, dadurch gekennzeichnet, daß das Bruchstück der heterologen DNA an der HindIII-Stelle des Plasmids ptrpED-50 eingebaut wird.

4. Verfahren zur Herstellung eines Mikroorganismus, der zur Expression von menschlichem Wachstumshormonvorläufer fähig ist, dadurch gekennzeichnet, daß man den Mikroorganismus mit Hilfe eines Übertragungsvektors transformiert, den man nach dem Verfahren nach einem der vorhergehenden Ansprüche erhalten hat.

5. Verfahren nach Anspruch 4, dadurch gekennzeichnet, daß der Mikroorganismus Escherichia coli ist.

6. Verfahren zur Synthese eines chimären Proteins, bestehend aus der Aminosäuresequenz des menschlichen Wachstumshormonvorläufers als C-terminale Sequenz, und einem Teil eines prokaryotischen Proteins als N-terminale Sequenz, gekennzeichnet durch die Inkubation eines durch das Verfahren von Anspruch 4 oder 5 gewonnenen Mikroorganismus.

7. Verfahren nach Anspruch 6, worin der prokaryotische Teil von einem Teil des trpD-Gens von Escherichia coli kodiert wird.

8. Verfahren zum Klonieren einer Desoxynukleotidsequenz nach Anspruch 1, welche für menschlichen Wachstumshormonvorläufer kodiert aus Hypophysengewebe, erhalten auf chirurgischem Wege bei einem einzigen Menschen, umfassend die folgenden Stufen:

a) Extraktion von für menschlichen Wachstumshormonvorläufer kodierender mRNA aus einer einzigen menschlichen Hypophyse,

c) Synthese einer einsträngigen cDNA mit einer der Nukleotidsequenz der mRNA komplementären Sequenz durch eine Reihe von Verfahren, darunter Inkubation mit einem Enzym, Extraktion zwecks Abtrennen von Proteinen aus dem Reaktionsgemisch, Äthanolfällung und basische Hydrolyse, wobei sämtliche besagte Verfahren in demselben Reaktionsröhrchen in Abwesenheit zugesetzter Träger-DNA durchgeführt werden,

d) chromatographische Reinigung der einsträngigen cDNA, wobei die die einsträngige DNA enthaltende Fraktionen mittels einer das Reaktionsprodukt nicht verbrauchenden Radiobestimmungsmethode nachgewiesen werden,

e) Synthese der doppelsträngigen cDNA mit einem Strang, dessen Nukleotidsequenz der mRNA entspricht, durch eine Reihe von Verfahren, darunter Inkubation mit einem Enzym und Extraktion zwecks Abtrennen von Proteinen, wobei besagte Verfahren einem einzigen Reaktionsrörchen in Abwesenheit zugesetzter Träger-DNA durchgeführt werden,

f) chromatographische Reinung der doppelsträngigen cDNA wie bei Stufe d),

g) weitere Behandlung der doppelsträngigen cDNA mit Enzym, im wesentlichen wie bei Stufen e) und f) beschrieben, um zur Insertion der cDNA in einem DNA-Übertragungsvektor geeignete Endgruppen bereitzustellen,

h) Insertion der doppelsträngigen cDNA in einen DNA-Übertragungsvektor, wobei ein rekombinanter Übertragungsvektor gebildet wird,

i) Transformation eines Mikroorganismus mit einem rekombinanten Übertragungsvektor, und

j) Selektion eines Mikroorganismusstamms, der von einer einzigen von einem rekombinanten Übertragungsvektor mit für menschlichen Wachstumshormonvorläufer kodierender cDNA transformierten Zelle abstammt, wobei eine für menschlichen Wachstumshormonvorläufer kodierende Desoxynukleotidsequenz kloniert wird.

**Revendications** pour les Etats Contractants BE, CH, DE, FR, GB, IT, LI, LU, NL, SE

1. Vecteur de transfert d'ADN comprenant une séquence de désoxynucléotides codant pour la pré-hormone de croissance humaine, comprenant:

5'-GGATCCTGTGGACAGCTCACCTAGCTGCAA $TG_{-26}$ $GCL_{-25}$ $ACL_{-24}$ $GGL_{-23}$ $QR_{-22}$ $S_{-22}$ $W_{-21}$ $GZ_{-21}$ $ACL_{-20}$ $QR_{-19}$ $S_{-19}$ $X_{-18}$ $TY_{-18}$ $X_{-17}$ $TX_{-17}$ $X_{-16}$ $TY_{-16}$ $GCL_{-15}$ $TTK_{-14}$ $GGL_{-13}$ $X_{-12}$ $TY_{-12}$ $X_{-11}$ $TY_{-11}$ $TGK_{-10}$ $X_{-9}$ $TY_{-9}$ $CCL_{-8}$ $TGG_{-7}$ $X_{-6}$ $CAJ_{-5}$ $GAJ_{-4}$ $GCL_{-3}$ $GTL_{-2}$ $CCL_{-1}$ $TTK_1$ $CCL_2$ $ACL_3$ $ATM_4$ $CCL_5$ $X_6$ $TY_6$ $QR_7$ $S_7$ $W_8$ $GZ_8$ $X_9$ $TY_9$ $TTK_{10}$ $GAK_{11}$ $AAK_{12}$ $GCL_{13}$ $ATG_{14}$ $X_{15}$ $TY_{15}$ $W_{16}$ $GZ_{16}$ $GCL_{17}$ $CAK_{18}$ $W_{19}$ $GZ_{19}$ $X_{20}$ $TY_{20}$ $CAK_{21}$ $CAJ_{22}$ $X_{23}$ $TY_{23}$ $GCL_{24}$ $TTK_{25}$ $GAK_{26}$ $ACL_{27}$ $TAK_{28}$ $CAJ_{29}$ $GAJ_{30}$ $TTK_{31}$ $GAJ_{32}$ $GAJ_{33}$ $ACL_{34}$ $TAK_{35}$ $ATM_{36}$ $CCL_{37}$ $AAJ_{38}$ $GAJ_{39}$ $CAJ_{40}$ $AAJ_{41}$ $TAK_{42}$ $QR_{43}$ $S_{43}$ $TTK_{44}$ $X_{45}$ $TY_{45}$ $CAJ_{46}$ $AAK_{47}$ $CCL_{48}$ $CAJ_{49}$ $ACL_{50}$ $QR_{51}$ $S_{51}$ $X_{52}$ $TY_{52}$ $TGK_{53}$ $TTK_{54}$ $QR_{55}$ $S_{55}$ $GAJ_{56}$ $QR_{57}$ $S_{57}$ $ATM_{58}$ $CCL_{59}$ $ACL_{60}$ $CCL_{61}$ $QR_{62}$ $S_{62}$ $AAK_{63}$ $W_{64}$ $GZ_{64}$ $GAJ_{65}$ $GAJ_{66}$ $ACL_{57}$ $CAJ_{68}$ $CAJ_{69}$ $AAJ_{70}$ $QR_{71}$ $S_{71}$ $AAK_{72}$ $X_{73}$ $TY_{73}$ $GAJ_{74}$ $X_{75}$ $TY_{75}$ $X_{76}$ $TY_{76}$ $W_{77}$ $GZ_{77}$ $ATM_{78}$ $QR_{79}$ $S_{79}$ $X_{80}$ $TY_{80}$ $X_{81}$ $TY_{81}$ $X_{82}$ $TY_{82}$ $ATM_{83}$ $CAJ_{84}$ $QR_{85}$ $S_{85}$ $TGGX_{87}$ $TY_{87}$ $GAJ_{88}$ $CCL_{89}$ $GTL_{90}$ $CAJ_{91}$ $TTK_{92}$ $X_{93}$ $TY_{93}$ $W_{94}$ $GZ_{94}$ $QR_{95}$ $S_{95}$ $GTL_{96}$ $TTK_{97}$ $GCL_{98}$ $AAK_{99}$ $AAK_{100}$ $X_{101}$ $TY_{101}$ $GTL_{102}$ $TAK_{103}$ $GGL_{104}$ $GCL_{105}$ $QR_{106}$ $S_{106}$ $GAK_{107}$ $QR_{108}$ $S_{108}$ $AAK_{109}$ $GTL_{110}$ $TAK_{111}$ $GAK_{112}$ $X_{113}$ $TY_{113}$ $X_{114}$ $TY_{114}$ $AAJ_{115}$ $GAK_{116}$ $X_{117}$ $TY_{117}$ $GAJ_{118}$ $GAJ_{119}$ $GGL_{120}$ $ATM_{121}$ $CAJ_{122}$ $ACL_{123}$ $X_{124}$ $TY_{124}$ $ATGGGL_{126}$ $W_{127}$ $GZ_{127}$ $X_{128}$ $TY_{128}$ $GAJ_{129}$ $GAK_{130}$ $GGL_{131}$ $QR_{132}$ $S_{132}$ $CCL_{133}$ $W_{134}$ $GZ_{134}$ $ACL_{135}$ $GGL_{136}$ $CAJ_{137}$ $ATM_{138}$ $TTK_{139}$ $AAJ_{140}$ $CAJ_{141}$ $ACL_{142}$ $TAK_{143}$ $QR_{144}$ $S_{144}$ $AAJ_{145}$ $TTK_{146}$ $GAK_{147}$ $ACL_{148}$ $AAK_{149}$ $QR_{150}$ $S_{150}$ $CAK_{151}$ $AAK_{152}$ $CAK_{153}$ $GAK_{154}$ $GCL_{155}$ $X_{156}$ $TY_{156}$ $X_{157}$ $TY_{157}$ $AAJ_{158}$ $AAK_{159}$ $TAK_{160}$ $GGL_{161}$ $X_{162}$ $TY_{162}$ $X_{163}$ $TY_{163}$ $TAK_{164}$ $TGK_{165}$ $TTK_{166}$ $W_{167}$ $GZ_{167}$ $AAJ_{168}$ $GAK_{169}$ $ATGGAK_{171}$ $AAJ_{172}$ $GTL_{173}$ $GAJ_{174}$ $ACL_{175}$ $TTK_{176}$ $X_{177}$ $TY_{177}$ $W_{178}$ $GZ_{178}$ $ATM_{179}$ $GTL_{180}$ $CAJ_{181}$ $TGK_{182}$

W$_{183}$   GZ$_{183}$   QR$_{184}$   S$_{184}$   GTL$_{185}$   GAJ$_{186}$   GGL$_{187}$   QR$_{188}$   S$_{188}$   TGK$_{189}$   GGL$_{190}$   TTK$_{191}$
TAGCTGCCCGGGTGGCATCCCTGTGACCCCTCCCCAGTGCCT
CTCCTGGCCCTGGAAGTTGCCACTCCAGTGCCCACCAGCCTTGTCCTAATAAAATTAAGT TGCATCpolyA-3'

dans laquelle
A est la désoxyadényle,
G est la désoxyguanyle,
C est le désoxycytosyle,
T est le thymidyle,
J est A ou G;
K est T ou C;
L est A, TC ou G;
M est A, C ou T;
X$_n$ est T ou C si Y$_n$ est A ou G, et C si Y$_n$ est C ou T;
Y$_n$ est A, G, C ou T si X$_n$ est C, et A ou G si X$_n$ est T;
W$_n$ est C ou A si Z$_n$ est G ou A, et C si Z$_n$ est C ou T;
Z$_n$ est A, G, C ou T si W$_n$ est C, et A ou G si W$_n$ est A;
QR$_n$ est TC si S$_n$ est A, G, C ou T, et AG si S$_n$ est T ou C;
S$_n$ est A, G, C ou T si QR$_n$ est TC, et T ou C si QR$_n$ est AG

et les nombres en indice, n, se rapportent à la position, dans la séquence d'acides aminés de l'hormone de croissance humaine, à laquelle correspond chaque triplet dans la séquence de nucléotides, selon le code génétique, les positions des acides aminés étant numérotées à partir de l'extrémité amino.

2. Micro-organisme transformé par le vecteur de transfert selon la revendication 1, et capable d'exprimer la pré-hormone de croissance humaine.

3. Plasmide pBR322, contenant au niveau de son site *Hind*III une séquence d'ADN telle que décrite dans la revendication 1.

4. Micro-organisme transformé par le plasmide de la revendication 3, et capable d'exprimer la pré-hormone de croissance humaine.

5. Plasmide p*trp*ED50, contenant au niveau de son site *Hind*III une séquence d'ADN telle que décrite dans la revendication 1.

6. Micro-organisme transformé par le plasmide de la revendication 5, et capable d'exprimer la pré-hormone de croissance humaine.

7. *Escherichia coli* transformé par le plasmide de la revendication 5 et capable d'exprimer la pré-hormone de croissance humaine.

8. Protéine chimère comprenant en tant que sa séquence C-terminale la séquence d'acides aminés de la pré-hormone de croissance humaine, et en tant que sa séquence N-terminale une partie d'une protéine de procaryote, telle qu'exprimée par un micro-organisme selon la revendication 6 ou la revendication 7.

9. Protéine selon la revendication 8, dans laquelle la partie de procaryote est codée par une partie du gène *trp*D de *Escherichia coli*.

10. Procédé pour le clonage d'une séquence de désoxynucléotides telle que décrite dans la revendication 1, codant pour la pré-hormone de croissance humaine provenant de tissu de glande pituitaire d'un sujet humain, obtenu par chirurgie, comprenant les étapes

a) d'extraction d'ARNm codant pour la pré-hormone de croissance humaine provenant d'une glande pituitaire d'un sujet humain,

b) de séparation de l'ARNm pratiquement exempt de protéine, d'ADN et d'autre ARN,

c) de synthèse d'un ADNc monocaténaire comportant une séquence de nucléotides complémentaire de celle de l'ARNm, dans une série d'opérations comprenant l'incubation avec une enzyme, une extraction pour l'élimination des protéines hors du mélange réactionnel, une précipitation à l'alcool et une hydrolyse alcaline, lesdites opérations étant toutes effectuées dans le même tube réactionnel, en l'absence d'ADN vecteur ajouté,

d) de purification de l'ADNc monocaténaire par chromatographie, les fractions contenant l'ADN monocaténaire étant localisées par un dosage radioactif qui ne consomme pas le produit de réaction,

e) de synthèse d'ADNc bicaténaire comportant un brin possédant une séquence de nucléotides correspondant à celle de l'ARNm, dans une série d'opérations comprenant l'incubation avec une enzyme et une extraction pour l'élimination des protéines, lesdites opérations étant effectuées dans un tube réactionnel unique, en l'absence d'ADN vecteur ajouté,

f) de purification de l'ADNc bicaténaire par chromatographie comme dans l'étape d),

g) de traitement ultérieur de l'ADNc bicaténaire avec une enzyme, essentiellement comme décrit dans les étapes e) et f), pour l'obtentiom de groupes terminaux appropriés pour l'insertion de l'ADNc dans un vecteur de transfert d'ADN,

h) d'insertion de l'ADNc bicaténaire dans un vecteur de transfert d'ADN, ce par quoi on obtient un vecteur de transfert recombinant,

i) de transformation d'un micro-organisme avec le vecteur de transfert recombinant, et

j) de sélection d'une souche de micro-organisme dérivée d'une cellule transformée unique, transformée par un vecteur de transfert recombinant contenant de l'ADNc codant pour la pré-hormone de croissance humaine, ce par quoi on réalise le clonage d'une séquence de désoxynucléotides codant pour la pré-hormone de

croissance humaine.

**Revendications** pour l'Etat Contractant AT

1. Procédé pour la modification d'un vecteur de transfert d'ADN, comprenant l'insertion, dans la séquence d'ADN dudit vecteur de transfert, d'un fragment d'ADN hétérologue comportant la séquence suivante:

5'-GGATCCTGTGGACAGCTCACCTAGCTGCAA $TG_{-26}$ $GCL_{-25}$ $ACL_{-24}$ $GGL_{-23}$ $QR_{-22}$ $S_{-22}$ $W_{-21}$ $GZ_{-21}$ $ACL_{-20}$ $QR_{-19}$ $S_{-19}$ $X_{-18}$ $TY_{-18}$ $X_{-17}$ $TX_{-17}$ $X_{-16}$ $TY_{-16}$ $GCL_{-15}$ $TTK_{-14}$ $GGL_{-13}$ $X_{-12}$ $TY_{-12}$ $X_{-11}$ $TY_{-11}$ $TGK_{-10}$ $X_{-9}$ $TY_{-9}$ $CCL_{-8}$ $TGG_{-7}$ $X_{-6}$ $CAJ_{-5}$ $GAJ_{-4}$ $GCL_{-3}$ $GTL_{-2}$ $CCL_{-1}$ $TTK_1$ $CCL_2$ $ACL_3$ $ATM_4$ $CCL_5$ $X_6$ $TY_6$ $QR_7$ $S_7$ $W_8$ $GZ_8$ $X_9$ $TY_9$ $TTK_{10}$ $GAK_{11}$ $AAK_{12}$ $GCL_{13}$ $ATG_{14}$ $X_{15}$ $TY_{15}$ $W_{16}$ $GZ_{16}$ $GCL_{17}$ $CAK_{18}$ $W_{19}$ $GZ_{19}$ $X_{20}$ $TY_{20}$ $CAK_{21}$ $CAJ_{22}$ $X_{23}$ $TY_{23}$ $GCL_{24}$ $TTK_{25}$ $GAK_{26}$ $ACL_{27}$ $TAK_{28}$ $CAJ_{29}$ $GAJ_{30}$ $TTK_{31}$ $GAJ_{32}$ $GAJ_{33}$ $ACL_{34}$ $TAK_{35}$ $ATM_{36}$ $CCL_{37}$ $AAJ_{38}$ $GAJ_{39}$ $CAJ_{40}$ $AAJ_{41}$ $TAK_{42}$ $QR_{43}$ $S_{43}$ $TTK_{44}$ $X_{45}$ $TY_{45}$ $CAJ_{46}$ $AAK_{47}$ $CCL_{48}$ $CAJ_{49}$ $ACL_{50}$ $QR_{51}$ $S_{51}$ $X_{52}$ $TY_{52}$ $TGK_{53}$ $TTK_{54}$ $QR_{55}$ $S_{55}$ $GAJ_{56}$ $QR_{57}$ $S_{57}$ $ATM_{58}$ $CCL_{59}$ $ACL_{60}$ $CCL_{61}$ $QR_{62}$ $S_{62}$ $AAK_{63}$ $W_{64}$ $GZ_{64}$ $GAJ_{65}$ $GAJ_{66}$ $ACL_{67}$ $CAJ_{68}$ $CAJ_{69}$ $AAJ_{70}$ $QR_{71}$ $S_{71}$ $AAK_{72}$ $X_{73}$ $TY_{73}$ $GAJ_{74}$ $X_{75}$ $TY_{75}$ $X_{76}$ $TY_{76}$ $W_{77}$ $GZ_{77}$ $ATM_{78}$ $QR_{79}$ $S_{79}$ $X_{80}$ $TY_{80}$ $X_{81}$ $TY_{81}$ $X_{82}$ $TY_{82}$ $ATM_{83}$ $CAJ_{84}$ $QR_{85}$ $S_{85}$ $TGGX_{87}$ $TY_{87}$ $GAJ_{88}$ $CCL_{89}$ $GTL_{90}$ $CAJ_{91}$ $TTK_{92}$ $X_{93}$ $TY_{93}$ $W_{94}$ $GZ_{94}$ $QR_{95}$ $S_{95}$ $GTL_{96}$ $TTK_{97}$ $GCL_{98}$ $AAK_{99}$ $AAK_{100}$ $X_{101}$ $TY_{101}$ $GTL_{102}$ $TAK_{103}$ $GGL_{104}$ $GCL_{105}$ $QR_{106}$ $S_{106}$ $GAK_{107}$ $QR_{108}$ $S_{108}$ $AAK_{109}$ $GTL_{110}$ $TAK_{111}$ $GAK_{112}$ $X_{113}$ $TY_{113}$ $X_{114}$ $TY_{114}$ $AAJ_{115}$ $GAK_{116}$ $X_{117}$ $TY_{117}$ $GAJ_{118}$ $GAJ_{119}$ $GGL_{120}$ $ATM_{121}$ $CAJ_{122}$ $ACL_{123}$ $X_{124}$ $TY_{124}$ $ATGGGL_{126}$ $W_{127}$ $GZ_{127}$ $X_{128}$ $TY_{128}$ $GAJ_{129}$ $GAK_{130}$ $GGL_{131}$ $QR_{132}$ $S_{132}$ $CCL_{133}$ $W_{134}$ $GZ_{134}$ $ACL_{135}$ $GGL_{136}$ $CAJ_{137}$ $ATM_{138}$ $TTK_{139}$ $AAJ_{140}$ $CAJ_{141}$ $ACL_{142}$ $TAK_{143}$ $QR_{144}$ $S_{144}$ $AAJ_{145}$ $TTK_{146}$ $GAK_{147}$ $ACL_{148}$ $AAK_{149}$ $QR_{150}$ $S_{150}$ $CAK_{151}$ $AAK_{152}$ $CAK_{153}$ $GAK_{154}$ $GCL_{155}$ $X_{156}$ $TY_{156}$ $X_{157}$ $TY_{157}$ $AAJ_{158}$ $AAK_{159}$ $TAK_{160}$ $GGL_{161}$ $X_{162}$ $TY_{162}$ $X_{163}$ $TY_{163}$ $TAK_{164}$ $TGK_{165}$ $TTK_{166}$ $W_{167}$ $GZ_{167}$ $AAJ_{168}$ $GAK_{169}$ $ATGGAK_{171}$ $AAJ_{172}$ $GTL_{173}$ $GAJ_{174}$ $ACL_{175}$ $TTK_{176}$ $X_{177}$ $TY_{177}$ $W_{178}$ $GZ_{178}$ $ATM_{179}$ $GTL_{180}$ $CAJ_{181}$ $TGK_{182}$ $W_{183}$ $GZ_{183}$ $QR_{184}$ $S_{184}$ $GTL_{185}$ $GAJ_{186}$ $GGL_{187}$ $QR_{188}$ $S_{188}$ $TGK_{189}$ $GGL_{190}$ $TTK_{191}$ TAGCTGCCCGGGTGGCATCCCTGTGACCCCTCCCCAGTGCCT CTCCTGGCCCTGGAAGTTGCCACTCCAGTGCCCACCAGCCTTGTCCTAATAAAATTAAGT TGCATCpolyA-3'

dans laquelle
A est la désoxyadényle,
G est la désoxyguanyle,
C est le désoxycytosyle,
T est le thymidyle,
J est A ou G;
K est T ou C;
L est A, TC ou G;
M est A, C ou T;
$X_n$ est T ou C si $Y_n$ est A ou G, et C si $Y_n$ est C ou T;
$Y_n$ est A, G, C ou T si $X_n$ est C, et A ou G si $X_n$ est T;
$W_n$ est C ou A si $Z_n$ est G ou A, et C si $Z_n$ est C ou T;
$Z_n$ est A, G, C ou T si $W_n$ est C, et A ou G si $W_n$ est A;
$QR_n$ est TC si $S_n$ est A, G, C ou T, et AG si $S_n$ est T ou C;
$S_n$ est A, G, C ou T si $QR_n$ est TC, et T ou C si $QR_n$ est AG et
les nombres en indice, n, se rapportent à la position, dans la séquence d'acides aminés de l'hormone de croissance humaine, à laquelle correspond chaque triplet dans la séquence de nucléotides, selon le code génétique, les positions des acides aminés étant numérotées à partir de l'extrémité amino.

2. Procédé selon la revendication 1, dans lequel le fragment d'ADN hétérologue est inséré au niveau du site *Hin*dIII du plasmide pBR322.

3. Procédé selon la revendication 1, dans lequel le fragment d'ADH hétérologue est inséré au niveau du site *Hin*dIII du plasmide p*trp*ED-50.

4. Procédé pour la production d'un micro-organisme capable d'exprimer la pré-hormone de croissance humaine, comprenant la transformation dudit micro-organisme avec un vecteur de transfert obtenu par le procédé selon l'une quelconque des revendications précédentes.

5. Procédé selon la revendication 4, dans lequel le micro-organisme est *Escherichia coli*.

6. Procédé pour la synthèse d'une protéine chimère comprenant en tant que sa séquence C-terminale la séquence d'acides aminés de la pré-hormone de croissance humaine, et en tant que sa séquence N-terminale une partie d'une protéine de procaryote, ledit procédé comprenant l'incubation d'un micro-organisme obtenu par le procédé selon la revendication 4 ou la revendication 5.

7. Procédé selon la revendication 6, dans lequel la partie de procaryote est codée par une partie du gène trpD,de *Escherichia coli*.

8. Procédé pour le clonage d'une séquence de désoxynucléotides telle que décrite dans la revendication 1, codant pour la pré-hormone de croissance humaine provenant de tissu, de glande pituitaire d'un sujet humain, obtenu par chirurgie, comprenant les étapes
a) d'extraction d'ARNm codant pour la pré-hormone de croissance humaine provenant d'une glande pituitaire d'un sujet humain,

b) de séparation de l'ARNm pratiquement exempt de protéine, d'ADN et d'autre ARN,

c) de synthèse d'un ADNc monocaténaire comportant une séquence de nucléotides complémentaire de celle de l'ARNm, dans une série d'opérations comprenant l'incubation avec une enzyme, une extraction pour l'élimination des protéines hors du mélange réactionnel, une précipitation à l'alcool et une hydrolyse alcaline, lesdites opérations étant toutes effectuées dans le même tube réactionnel, en l'absence d'ADN vecteur ajouté,

d) de purification de l'ADNc monocaténaire par chromatographie, les fractions contenant l'ADN monocaténaire étant localisées par un dosage radioactif qui ne consomme pas le produit de réaction,

e) de synthèse d'ADNc bicaténaire comportant un brin possédant une séquence de nucléotides correspondant à celle de l'ARNm, dans une série d'opérations comprenant l'incubation avec une enzyme et une extraction pour l'élimination des protéines, lesdites opérations étant effectuées dans un tube réactionnel unique, en l'absence d'ADN vecteur ajouté,

f) de purification de l'ADNc bicaténaire par chromatographie comme dans l'étape d),

g) de traitement ultérieur de l'ADNc bicaténaire avec une enzyme, essentiellement comme décrit dans les étapes e) et f), pour l'obtention de groupes terminaux appropriés pour l'insertion de l'ADNc dans un vecteur de transfert d'ADN,

h) d'insertion de l'ADNc bicaténaire dans un vecteur de transfert d'ADN, ce par quoi on obtient un vecteur de transfert recombinant,

i) de transformation d'un micro-organisme avec le vecteur de transfert recombinant, et,

j) de sélection d'une souche de micro-organisme dérivée d'une cellule transformée unique, transformée par un vecteur de transfert recombinant contenant de l'ADNc codant pour la pré-hormone de croissance humaine, ce par quoi on réalise le clonage d'une séquence de désoxynucléotides codant pour la pré-hormone de croissance humaine.

pre-GH →

1  2  3  4  5  6  a

HUMAN

FIG. I

a b c d e f g h i j k

FIG. 2

FIG. 3

FIG. 4